# FASCICULE DE BREVET EUROPEEN

(11) **EP 1 969 651 B1**
(45) Date de publication et mention de la délivrance du brevet: **16.09.2009**
(21) Numéro de dépôt: 06841607.2
(22) Date de dépôt: 22.12.2006
(51) Int. Cl.: H01L 51/30, C07D 213/26, C07D 213/30

(54) **COMPLEXES SENSIBILISATEURS, LEUR PROCEDE DE PREPARATION, MATERIAU HYBRIDE INORGANIQUE-ORGANIQUE SEMICONDUCTEUR LES COMPRENANT, ET CELLULE PHOTOVOLTAIQUE COMPRENANT CE MATERIAU**
SENSIBILISIERUNG VON KOMPLEXEN, HERSTELLUNGSVERFAHREN DAFÜR, HYBRIDES ANORGANISCHES/ORGANISCHES HALBLEITERMATERIAL MIT IHNEN UND DIESES MATERIAL UMFASSENDE FOTOVOLTAISCHE ZELLE
SENSITIZING COMPLEXES, THEIR METHOD OF PRODUCTION, HYBRID INORGANIC/ ORGANIC SEMICONDUCTOR MATERIAL COMPRISING THEM, AND PHOTOVOLTAIC CELL COMPRISING THIS MATERIAL

(30) Priorité: 23.12.2005 FR 0513259
(43) Date de publication de la demande: 17.09.2008
(73) Titulaire: Commissariat à l'Energie Atomique, 75015 Paris (FR)
(72) Inventeur: BUVAT, Pierrick, F-37250 Montbazon (FR); ODOBEL, Fabrice, F-44300 Nantes (FR); HOUARNER, Coralie, F-44300 Nantes (FR); BLART, Errol, F-44119 Grandchamp des Fontaines (FR)
(74) Mandataire: Ilgart, Jean-Christophe
(86) Numéro de dépôt international: PCT/EP2006/070189
(87) Numéro de publication internationale: WO 2007/071792

(56) Documents cités:
- FR-A- 2 862 429
- KREBS ET AL: "Dye sensitized photovoltaic cells: Attaching conjugated polymers to zwitterionic ruthenium dyes" SOLAR ENERGY MATERIALS AND SOLAR CELLS, ELSEVIER SCIENCE PUBLISHERS, AMSTERDAM, NL, vol. 90, no. 2, 23 janvier 2006 (2006-01-23), pages 142-165, XP005170973 ISSN: 0927-0248
- GALOPPINI E: "Linkers for anchoring sensitizers to semiconductor nanoparticles" COORDINATION CHEMISTRY REVIEWS, ELSEVIER SCIENCE, AMSTERDAM, NL, vol. 248, no. 13-14, juillet 2004 (2004-07), pages 1283-1297, XP004578920 ISSN: 0010-8545
- KIMURA, MUTSUMI ET AL: "Synthesis of Multicomponent Systems Composed of One Phthalocyanine and Four Terpyridine Ligands" INORGANIC CHEMISTRY , 40(18), 4775-4779 CODEN: INOCAJ; ISSN: 0020-1669, 31 juillet 2001 (2001-07-31), XP002419363

## Description

### DOMAINE TECHNIQUE

La présente invention concerne de nouveaux complexes sensibilisateurs, colorants, destinés à être mis en oeuvre dans les cellules photoélectrochimiques, et plus particulièrement dans les cellules photovoltaïques.

La présente invention concerne également un procédé de préparation de ces nouveaux complexes sensibilisateurs, colorants.

La présente invention concerne en outre un matériau hybride inorganique-organique semiconducteur P-N comprenant lesdits complexes sensibilisateurs, colorants, et un substrat en céramique oxyde poreuse tel que le TiO₂.

La présente invention concerne aussi un procédé de préparation dudit matériau hybride inorganique-organique.

Enfin, l'invention a trait à une cellule photovoltaïque comprenant ledit matériau hybride inorganique-organique.

Le domaine technique de l'invention peut être défini de manière générale comme celui des cellules photo-électrochimiques, plus particulièrement des cellules photovoltaïques ou encore des diodes électroluminescentes.

### ETAT DE LA TECHNIQUE ANTERIEURE

Une cellule photovoltaïque est un dispositif permettant la conversion d'une énergie photochimique en énergie électrique.

Généralement, une cellule photovoltaïque se compose de matériaux semi-conducteurs dopés P (c'est-à-dire présentant un déficit d'électrons, c'est-à-dire des trous de charges) et de matériaux semi-conducteurs dopés N (c'est-à-dire présentant un excès d'électrons), réunis par une jonction dite « jonction P-N », qui permet une séparation entre les électrons et les trous de charge. Cette séparation génère une différence de potentiel à la jonction P-N et par conséquent un courant électrique si l'on place un contact sur la zone N et un contact sur la zone P et une résistance (à savoir un dispositif destiné à être alimenté en courant électrique) entre ces deux contacts.

Ainsi, lorsque la lumière frappe la zone de la cellule constituée de la jonction entre le matériau semi-conducteur de type P et le matériau semi-conducteur de type N, les photons constitutifs de la lumière sont absorbés par ladite zone et chaque photon absorbé donne naissance à un électron et un trou (on parle de paire électron-trou), ladite paire étant séparée à la jonction du matériau de type N et du matériau du type P, créant ainsi une différence de potentiel de part et d'autre de cette jonction.

Jusqu'à récemment, la plupart des cellules photovoltaïques ont été fabriquées à partir de silicium, plus précisément de silicium dopé par des atomes tels que du phosphore pour constituer la zone N et de silicium dopé par des atomes tels que du bore pour constituer la zone P de la cellule. Toutefois, l'utilisation du silicium s'avère coûteuse.

Pour remédier à cet inconvénient, la recherche s'est attachée à développer de nouveaux matériaux pouvant entrer dans la constitution de cellules photovoltaïques.

Ainsi, des cellules photovoltaïques ont été conçues à partir d'un matériau semi-conducteur de type P-N comprenant une zone semi-conductrice N solide et une zone semi-conductrice P liquide. Plus précisément, la zone semi-conductrice N est constituée d'une céramique oxyde poreuse, par exemple du dioxyde de titane dont les pores sont remplis d'un électrolyte liquide conducteurs de charges, cet électrolyte remplissant, par analogie avec les cellules photovoltaïques classiques, le rôle de zone semi-conductrice P.

Ce type de cellule photovoltaïque dont le principe se fonde donc essentiellement sur la sensibilisation de couches minces d'oxyde de titane nanocristallines, permet d'atteindre des efficacités de photoconversion de l'ordre de 10% à l'aide de sensibilisateurs à base de complexes de ruthénium polypyridiniques [1] [2] [3].

Ce type de cellule photovoltaïque est notamment décrit dans la demande internationale de brevet WO-A-93/19479.

Toutefois, il a été constaté que les cellules photovoltaïques utilisant un électrolyte liquide présentent les inconvénients suivants :
- une faible stabilité dans le temps, liée à l'évaporation des solvants entrant dans la composition de l'électrolyte ;
- une plage de température de fonctionnement relativement limitée en raison du caractère volatil des solvants entrant dans la constitution de l'électrolyte ;
- un risque de précipitation des sels entrant dans la constitution de l'électrolyte, lorsque la cellule photovoltaïque est amenée à fonctionner à des températures très basses, telles que des températures de l'ordre de -10°C à -40°C.
- une mise en oeuvre contraignante du fait de l'utilisation d'un électrolyte liquide, excluant notamment l'emploi de supports organiques souples et/ou de grandes dimensions.

Autrement dit, le développement industriel à large échelle de ces cellules photovoltaïques à électrolyte liquide se heurte à un problème technologique majeur occasionné par l'électrolyte liquide que contient la cellule. En effet, l'électrolyte liquide qui est généralement constitué par une solution d'acétonitrile et de carbonate de propylène solubilisant le médiateur rédox (ioduré/iode) est un mélange chimiquement agressif qui rend difficile « l'enrobage étanche » des deux électrodes de la cellule.

Ce problème est même aggravé par le dégagement de gaz qui peut se produire à l'intérieur de la cellule.

La température de fonctionnement d'une cellule photovoltaïque excède souvent 50°C sous ensoleillement et la volatilité des solvants de l'électrolyte génère alors une surpression à l'intérieur du montage.

En outre, la vitesse de réduction de l'ion triiodure à la contre électrode est connue pour être l'étape limitante du cycle spectro-électrochimique [4]. En raison du caractère corrosif excessif de l'iode présent dans l'électrolyte liquide, il est impossible d'introduire des fils métalliques qui seraient encapsulés dans le conducteur transparent qui est généralement de l'oxyde mixte d'étain ou d'indium ou de l'oxyde d'étain dopé, et qui permettraient d'améliorer le transport de charges dans le circuit électrique extérieur. Ces fils conducteurs annexes, s'ils pouvaient être utilisés, s'avéreraient très bénéfiques pour drainer le courant de cellules photovoltaïques de grande surface.

On a donc pensé résoudre les problèmes liés à l'électrolyte liquide en utilisant un conducteur solide entre la photocathode et la contre-électrode. Ainsi, on pensait pouvoir éliminer les problèmes d'étanchéité et accroître la densité de courant échangée entre le sensibilisateur oxydé et la contre électrode.

Les travaux de recherche se sont focalisés sur la conception de cellules photovoltaïques comprenant des matériaux semi-conducteurs P-N, comprenant à la fois une zone semi-conductrice N solide, et une zone semi-conductrice P solide constituée de matériaux organiques.

Ainsi, la demande de brevet EP 1176646 décrit des cellules photovoltaïques comprenant une zone semi-conductrice N constituée d'une céramique oxyde de titane sensibilisée par des nanoparticules de semi-conducteur inorganique et comprenant une zone semi-conductrice P constituée par une molécule organique conducteur de trous appartenant à la famille des composés spiro- et hétérospiro, en particulier la molécule de 2,2',7,7'-tétrakis(N,N-di-p-méthoxyphénylamine)9,9'-spirobifluorène (connu sous l'abréviation OMeTAD). Cette zone P est obtenue par enduction centrifuge (ou « spin coating » selon la terminologie anglo-saxonne) de la zone N avec une solution comprenant l'OMeTAD et du chlorobenzène. Toutefois, le temps de contact entre la solution contenant de l'OMeTAD et la couche en oxyde de titane est relativement court du fait de l'évaporation rapide du chlorobenzène et de la méthode de dépôt utilisée. Ceci se traduit notamment par une interpénétration limitée des zones N et P, cette interpénétration limitée étant également due à la diffusion lente des molécules d'OMeTAD vers la surface interne de la céramique (à savoir la surface de paroi des pores). Cette interpénétration limitée des zones N et P se traduit par un rendement solaire faible.

La demande de brevet EP 0 917 208 décrit une cellule photovoltaïque comprenant un film photoactif constitué d'une matrice de polymère organique à base de polyparaphénylènevinylène (connu sous l'abréviation PPV) dans laquelle sont dispersées des nanoparticules de type semi-conducteur (en particulier TiO₂). Dans cette configuration, le PPV assure la fonction de conducteurs de trous (c'est-à-dire la fonction d'une zone semi-conductrice P) et la fonction d'une substance chromophore en absorbant les photons issus de la lumière tandis que les nanoparticules dispersées assurent le rôle de conducteur d'électrons (zone semi-conductrice N). Cependant, ce type de configuration présente les inconvénients suivants :
- la dispersion de nanoparticules dans la matrice organique limite la percolation de celles-ci et ainsi la conduction des électrons vers la couche collectrice d'électrons de la cellule photovoltaïque ;
- la dispersion de nanoparticules dans la matrice organique induit un fort taux de recombinaison électron-trou à l'interface PPV/nanoparticules.

La demande de brevet WO 93/20569 décrit une cellule photovoltaïque à colorant, comprenant une zone constituée par un film poreux d'oxyde de titane sensibilisé par une substance chromophore et une zone constituée d'un polymère conducteur de trous. Le procédé de fabrication de ce type de cellule photovoltaïque consiste à déposer à haute température (de l'ordre de 300°C) le polymère conducteur à l'état fondu sur le film poreux d'oxyde de titane. Toutefois, le matériau obtenu présente les inconvénients suivants:
- il se caractérise par une interpénétration entre le film poreux et le polymère limitée par la diffusion du polymère à l'état fondu dans la porosité du film d'oxyde de titane ;
- il comprend une jonction lâche entre le matériau semi-conducteur N et le matériau semi-conducteur P du fait que la liaison entre ces deux zones se fait par des interactions faibles de type Van der Waals ;
- l'opération réalisée à haute température (de l'ordre de 200 à 300°C) peut endommager la substance chromophore et empécher l'utilisation d'une large gamme de substances chromophores aux températures de décomposition basses.

Plusieurs autres publications concernent la même approche consistant à mettre en oeuvre un conducteur solide.

A cet effet, différents conducteurs solides ont été testés ; il s'agit de matériaux inorganiques tels que le CuI ([5] ou CuSSN [6] [7], de polymères organiques tels que le polypyrrole [8] [9] [10] ou le polythiophène [11] à [21], ou encore de petites molécules organiques telles que les amines aromatiques tertiaires [22] à [25]. Ces films organiques ou inorganiques jouent le rôle de transporteur de charges entre la contre-électrode et le sensibilisateur. La photoanode de TiO₂ est préalablement imprégnée du sensibilisateur puis elle est recouverte d'un film composé d'un matériau (molécule ou polymère) conducteur de l'électricité. Le résultat le plus intéressant est probablement celui publié par Grätzel [22]. Toutefois, aucun des systèmes décrits ne donne satisfaction puisque le rendement de la cellule chute de manière très importante par rapport à une cellule classique comportant un électrolyte liquide, cette chute est d'un facteur 20 dans le meilleur des cas.

On peut supposer que les performances limitées de ces cellules photovoltaïques et notamment leur chute de rendement sont dues notamment à l'un ou plusieurs des inconvénients suivants :
- une interpénétration limitée de la zone semi-conductrice N et de la zone semi-conductrice P ;
- un fort taux de recombinaison électron-trou à la jonction de ces zones du fait de la faible interpénétration des différents composants zone N/chromophore/zone P.

En d'autres termes, on peut penser que la chute de rendement de la cellule solide est due à un transfert électronique inefficace entre le sensibilisateur, colorant, et le conducteur électrique solide.

Cela pourrait s'expliquer par une mauvaise adsorption du semiconducteur P sur le sensibilisateur et aussi par un mauvais mouillage du réseau nanocristallin du semiconducteur par le conducteur électrique, lié à une diffusion peu profonde de ce matériau dans les pores du semiconducteur [11] [26].

Il existe donc de manière générale un besoin, au vu de ce qui précède, pour une cellule photovoltaïque présentant des performances améliorées et notamment un rendement accru par rapport aux cellules photovoltaïques à électrolyte solide de l'art antérieur.

Il existe aussi un besoin pour une cellule qui puisse être munie facilement de fils conducteurs immergés dans le conducteur transparent pour drainer les charges dans le circuit électrique externe.

Il existe en particulier un besoin pour un matériau semiconducteur de type P-N qui présente une interaction, une interpénétration, forte entre la zone semiconductrice P et la zone semiconductrice N tout en permettant toutefois de limiter les phénomènes de court-circuit entre les deux zones.

Il existe en d'autres termes un besoin pour accroître l'efficacité du transfert électronique entre le sensibilisateur, colorant, et le conducteur électrique solide et pour améliorer le mouillage du réseau nanocristallin du semiconducteur tel que le TiO₂ par le conducteur électrique.

Le but de la présente invention est de fournir un sensibilisateur, un matériau semiconducteur de type P-N et une cellule photovoltaïque qui répondent entre autres aux besoins énumérés ci-dessus.

Le but de la présente invention est également de fournir un sensibilisateur, un matériau semiconducteur de type P-N et une cellule photovoltaïque qui ne présentent pas les inconvénients, limitations, défauts et désavantages des sensibilisateurs, matériaux semiconducteurs de type P-N et cellules photovoltaïques de l'art antérieur et qui résolvent les problèmes qui se posent dans l'art antérieur.

### EXPOSÉ DE L'INVENTION

Ce but, et d'autres encore sont atteints, conformément à l'invention par un complexe de formule (I) 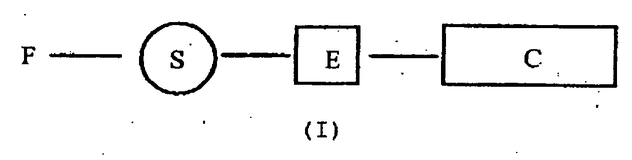 dans laquelle :
- F représente un ou plusieurs groupes aptes à se greffer chimiquement à un substrat en céramique oxyde poreuse semiconductrice ;
- S représente un groupe sensibilisateur d'une céramique oxyde poreuse semiconductrice ;
- C est un polymère conducteur de l'électricité ;
- E est un groupe espaceur déconjuguant permettant d'isoler électriquement le sensibilisateur (S) du polymère conducteur de l'électricité (C).

Lesdits complexes de formule (I) selon l'invention sont des composés nouveaux qui se différencient fondamentalement, notamment des composés sensibilisateurs de l'art antérieur. En effet, ces complexes présentent de manière totalement nouvelle une structure dans laquelle un groupe sensibilisateur S et un groupe conducteur sont reliés par l'intermédiaire d'une liaison covalente E non conjuguée.

De tels composés présentant une telle structure spécifique ne sont ni décrits ni suggérés dans l'art antérieur. De manière étonnante, les inventeurs ont associés dans une même molécule, à savoir le complexe de formule (I), un sensibilisateur S et un polymère conducteur C, et de plus, de manière encore plus surprenante, ils ont séparés S et C par un groupe espaceur déconjuguant.

Les nouveaux complexes selon l'invention jouent, de manière surprenante, à la fois le rôle de sensibilisateur et le rôle de polymère conducteur de l'électricité. Associer ces deux fonctions dans la même molécule est inattendu.

Les complexes de l'invention peuvent être définis comme des sensibilisateurs fonctionnalisés par une molécule permettant des transports de charges électriques : à savoir le polymère conducteur C.

Si la longueur de cette molécule est suffisante, comme c'est le cas des polymères C des complexes de l'invention, elle « sortira » des pores de la structure nanoporeuse du semiconducteur. Ainsi, tous les sensibilisateurs seront en contact électrique avec le matériau conducteur solide déposé à la surface de la photoanode.

Une autre caractéristique structurelle nouvelle importante des complexes selon l'invention est l'insertion d'une unité déconjuguante entre le sensibilisateur et le polymère conducteur qui permet de conserver les propriétés électroniques intrinsèques du sensibilisateur après sa fonctionnalisation par le polymère conducteur. Ainsi, le rendement d'injection de l'électron dans le semiconducteur depuis l'état excité du sensibilisateur est conservé après le greffage de la chaîne de polymère conducteur C.

Le greffage des nouveaux complexes selon l'invention - comprenant un groupe sensibilisateur lié par une liaison covalente non conjuguée à une chaîne d'un polymère conducteur - tel qu'un polythiophène ou un dérivé de celui-ci sur une électrode conductrice recouverte d'un semiconducteur n à large bande interdite tel que TiO₂, ZnO ou SnO₂, éventuellement suivi du dépôt d'un film de conducteur de charges p tel qu'un polymère conducteur choisi en particulier parmi les polythiophènes, les polypyrroles et leurs dérivés respectifs, ou de petites molécules organiques, et finalement du dépôt d'une électrode métallique par exemple en or, argent ou aluminium, conduit à des performances accrues de la photopile.

Le complexe sensibilisateur-polymère conducteur selon l'invention constitue un nouveau matériau qui permet d'améliorer les transferts.

Le document [31] divulgue des complexes de type F-S-C qui peuvent être adsorbés sur des électrodes d'anatase macroporeux. Les complexes décrits dans ce document ne comprennent pas comme les complexes de formule (I) selon l'invention de groupe espaceur déconjuguant E.

L'introduction d'un espaceur E conduit à de nouveaux composés fondamentalement différents de ceux qui sont rencontrés dans [31]. La présence d'un espaceur E joue un rôle fondamental dans les processus d'injection de charge, à l'origine des phénomènes photovoltaïques.

Le document [32] décrit un matériau semiconducteur P-N susceptible d'être obtenu par les étapes successives suivantes :
- fonctionnalisation d'une céramique oxyde poreuse semiconductrice, par greffage chimique d'un ou plusieurs composés comprenant un groupe susceptible d'être polymérisé avec un ou plusieurs précurseurs d'un polymère conducteur de l'électricité, et au moins un groupe apte à se greffer chimiquement audit subtrat ;
- imprégnation dudit substrat fonctionnalisé par une solution comprenant le ou lesdits précurseurs ;
- polymérisation desdits précurseurs.

Il est mentionné que ce matériau peut comprendre de manière facultative une ou plusieurs substances chromophores sensibilisant ladite céramique.

Il est précisé que cette substance peut être soit absorbée, soit greffée chimiquement à la surface et à l'intérieur du substrat en céramique oxyde.

Le chromophore n'est pas intégré dans le complexe (comme le groupe S de notre complexe) mais fixé directement à la surface et à l'intérieur du substrat en céramique.

Des groupes espaceurs correspondant aux groupes E des matériaux selon l'invention sont éventuellement présents dans les matériaux du document [32], c'est-à-dire ne sont pas obligatoirement présents dans les matériaux du document [32].

Il est en outre indiqué que le groupe espaceur (« E ») peut séparer le polymère des groupes aptes à se lier chimiquement : ce groupe « E » qui est optionnel ne sépare donc pas comme dans notre complexe le polymère du groupe sensibilisateur, chromophore.

Enfin, un complexe de formule (I) selon l'invention ne peut pas être préparé et ne peut pas exister en tant que tel dans le document [32] puisque l'on procède dans ce document à la préparation du matériau semiconducteur par des étapes de synthèse successives à partir de la céramique.

L'idée de base de notre invention consiste à concevoir des composés au sein desquels les espèces participant au transport des charges ne sont plus ni superposées, ni même mises en contact intime, mais liées physiquement par des liens covalents.

Cependant, nous avons établi que le greffage du polymère sur le sensibilisateur sans espaceur, tel que décrit dans [31],modifie de façon radicale les propriétés électroniques du sensibilisateur. En effet, dans ce cas, les niveaux électroniques des états excités du ligand par exemple terpyridinique portant le polymère conducteur sont abaissés de telle sorte que les électrons sont transférés vers le polymère conducteur et non plus vers le semiconducteur par exemple n, ce qui nuit fortement au rendement photovoltaïque (voir exemples joints).

Pour remédier à cet inconvénient, nous montrons selon l'invention qu'il faut introduire un lien déconjuguant entre le sensibilisateur et le polymère conducteur. Ceci a pour effet de conserver les propriétés électroniques du sensibilisateur tout en permettant un transfert des charges vers le polymère conducteur. Pour cela, il convient d'utiliser de préférence des liens faiblement déconjuguants tels que des fonctions -CH₂-CH₂- ou des cycles aromatiques substitués (torsion du cycle hors du plan de recouvrement des orbitales pi du fait de gènes stériques). De façon surprenante, l'introduction du lien déconjuguant ne nuit pas au transfert des charges vers le polymère conducteur.

L'introduction de l'espaceur déconjuguant a donc pour effet de restaurer les propriétés du sensibilisateur, tout en permettant le transfert des charges vers le polymère conducteur greffé.

Avant de décrire l'invention de manière plus détaillée, nous précisons les définitions suivantes :
Par substrat semiconducteur auquel est greffé le complexe de formule (I), on entend généralement la céramique oxyde poreuse qui fait partie d'un matériau hybride inorganique-organique semiconducteur P-N comprenant une céramique oxyde poreuse à laquelle est greffée chimiquement le complexe de formule (I).

Ledit matériau hybride inorganique-organique semiconducteur de type P-N est décrit plus loin ; il comprend une zone semiconductrice de type N et une zone semiconductrice de type P. Dans le cadre de l'invention, la zone semiconductrice de type N de préférence à large bande interdite peut être constituée par ledit substrat en céramique oxyde poreuse, auquel cas la zone semiconductrice de type P sera constituée par le ou les polymères conducteurs de l'électricité.

Ou bien, la zone conductrice de type N peut être constituée par le ou les polymères conducteurs de l'électricité, auquel cas la zone semiconductrice de type P dudit matériau hybride est constituée par le substrat en céramique oxyde poreuse.

On précise également que ce substrat peut se présenter sous forme d'un bloc (ou d'une pièce) ou encore sous forme d'un revêtement (par exemple d'un film présentant une épaisseur de 10 nm à 100 µm).

Par polymère conducteur de l'électricité, on entend un polymère présentant des propriétés de conduction électrique sans dopage (auquel cas le polymère sera un polymère conducteur intrinsèque de l'électricité) ou avec dopage (auquel cas le polymère sera un polymère conducteur extrinsèque de l'électricité), la conduction électrique étant véhiculée soit par le biais d'électrons (en ce qui concerne les polymères conducteurs de type N), soit par le biais de trous, qui correspondent à des 'espaces' laissés vacants par des électrons (en ce qui concerne les polymères conducteurs de type P). Des exemples spécifiques de ces différents types de polymères seront donnés ultérieurement.

Par greffage chimique, on entend, dans ce qui précède et ce qui suit, une immobilisation du ou des composés complexes de formule (I) sus-mentionnés sur le substrat susmentionné par le biais d'une liaison chimique covalente, voire iono-covalente. On précise que cette immobilisation se fait à la fois à la surface externe du substrat et également à la surface interne dudit substrat, c'est-à-dire sur la surface de parois des pores du sustrat. Il est bien entendu que le greffage chimique n'exclut pas l'existence d'interactions simples physiques telles que des interactions dites de Van der Waals ou des interactions de type de liaisons hydrogène entre les composés susmentionnés et le substrat susmentionné.

Par groupe apte à se greffer chimiquement audit substrat, on entend des groupes réagissant avec les groupes réactifs présents sur la céramique oxyde, tels que des groupes -OH, ces groupes -OH résultant d'un phénomène d'hydratation spontanée de la céramique, soit sous l'effet de l'humidité de l'atmosphère ambiante, soit sous l'effet d'une humidité provoquée pour créer ces groupes.

Le ou les groupes aptes à se greffer chimiquement à la céramique peuvent être choisis parmi les groupes de formules suivantes :
- COOR¹ avec R¹ représentant un atome d'hydrogène, un groupe alkyle comprenant de 1 à 30 atomes de carbone ou un groupe phényle ;
- COCl ;
- COCH₂CO-R¹ avec R¹ représentant un atome d'hydrogène, un groupe alkyle comprenant de 1 à 30 atomes de carbone ou un groupe phényle ;
- PO(OH)₂, -PO(OR²)(OH) ou -PO(OR²)(OR³) avec R² et R³, identiques ou différents, représentant un groupe alkyle comprenant de 1 à 30 atomes de carbone ou un groupe phényle ;
- CO(NHOH) ;
- M(OR⁴)ₘ₋ₓZₓ avec x étant un entier allant de 1 à (m-1), M étant un métal ou un métalloïde, m étant un degré d'oxydation de M, R⁴ représentant un atome d'hydrogène, un groupe alkyle comprenant de 1 à 30 atomes de carbone, un groupe phényle, un cation métallique monovalent, ou un groupe de formule N⁺R¹₄, avec R¹ représentant un atome d'hydrogène, un groupe alkyle comprenant de 1 à 30 atomes de carbone, ou un groupe phényle, et Z représente un atome d'hydrogène, un groupe alkyle comprenant de 1 à 30 atomes de carbone, un groupe phényle ou un atome d'halogène ;
- SO₃M' avec M' représentant un atome d'hydrogène, un cation métallique monovalent ou un groupe de formule N⁺R¹₄ avec R¹ représentant un atome d'hydrogène, un groupe alkyle comprenant de 1 à 30 atomes de carbone ou un groupe phényle ;
- B(OM')₂ avec M' représentant un atome d'hydrogène, un cation métallique monovalent ou un groupe de formule N⁺R¹₄ avec R¹ représentant un atome d'hydrogène, un groupe alkyle comprenant de 1 à 30 atomes de carbone ou un groupe phényle ;
- OH ;
et les combinaisons de ceux-ci.

Pour le groupe de formule -M(OR⁴)ₙ₋ₓZₓ telle que définie précédemment, M peut représenter un élément métallique, tel qu'un élément de transition de degré d'oxydation n donné ou un élément métalloïde tel que Al, Ga, In, Si de degré d'oxydation n donné, les degrés d'oxydation envisageables pour chaque élément métallique ou métalloïde étant connu de l'homme du métier. A titre d'exemple de groupe conforme à cette définition, on peut citer le groupe de formule -Si(OR⁴)₃₋ₓZₓ avec x étant un entier allant de 1 à 3.

Le greffage chimique avec le substrat en céramique oxyde poreuse se fait avantageusement par les groupes énumérés précédemment.

Le complexe selon l'invention comprend un groupe sensibilisateur S du substrat semiconducteur, par exemple du substrat semiconducteur de type N à large bande interdite, qui est la céramique oxyde.

Ce groupe peut encore être appelé groupe chromophore sensibilisant ladite céramique.

On précise que, selon l'invention, on entend par substance chromophore une substance apte à absorber une lumière dans le domaine IR, UV et visible et à libérer en contrepartie de cette absorption des électrons. Dans le cadre de l'invention, les électrons vont être captés soit par la céramique oxyde (si celle-ci est semi-conductrice N) soit par le ou les polymères conducteurs de l'électricité (si ceux-ci sont des polymères de type N), tandis que les trous de charges laissés par les électrons libérés sont captés soit par la céramique oxyde (si celle-ci est semi-conductrice de type P) soit par le ou les polymères conducteurs de l'électricité (si ceux-ci sont des polymères de type P).

Il est entendu qu'une substance chromophore donnée présente une sensibilité spectrale bien déterminée et que le choix de cette substance devra être adaptée à la source de lumière, afin de présenter un rendement d'absorption de lumière aussi performant que possible.

Ledit groupe sensibilisateur S peut être choisi par exemple parmi les complexes polypyridiniques avec un métal de transition et les cations organiques tels que les phtalocyanines, les coumarines, et les cyanines.

Avantageusement, ledit groupe sensibilisateur est un groupe de formule (II) ou de formule (IIA) :

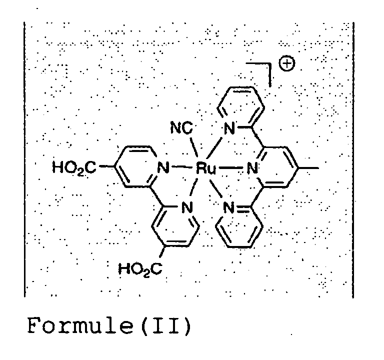

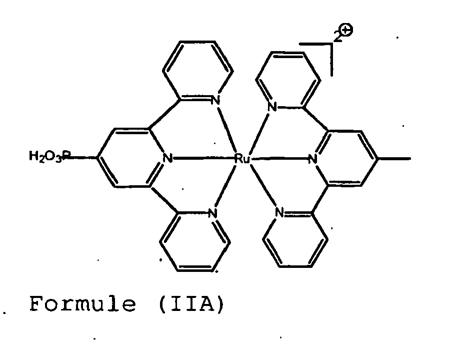

Le polymère conducteur de l'électricité C est choisi généralement parmi les poly(acétylène)s, les poly(p-phénylène)s, les poly(p-phénylène vinylène)s), les poly(p-phénylène sulfure)s, les poly(pyrrole)s, les poly(thiophène)s, les poly(alkyl thiophène)s, les poly(dialkylthiophène)s, les poly(alcoxy thiophènes), les poly(furane)s, les poly(azulène)s, les poly(azine)s, les poly(aniline)s, les poly(cyanophénylène vinylène)s, les poly(parapyridyl vinylène)s, les poly(dioxythiophènes)("PEDOT") dont le motif répétitif répond à la formule suivante : 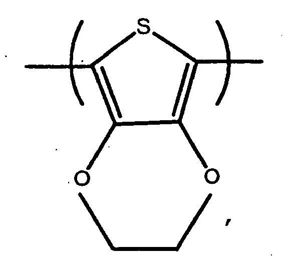 et les mélanges et/ou combinaisons et/ou copolymères de ceux-ci (c'est-à-dire les copolymères formés à partir des monomères constituant les polymères ci-dessus, entre eux, ou avec d'autres monomères).

Parmi cette liste de polymères, les polymères de type N sont les poly(cyanophénylène vinylène)s et les poly(p-pyridyl vinylène)s.

Parmi cette liste de polymère, les polymères de type P sont les poly(p-phénylène)s, les poly(p-phénylène vinylène)s, les poly(p-phénylènesulfures), les polypyrroles, les polythiophènes, les poly(alkylthiophènes) tels que le poly(3-octylthiophène), les poly(dialkylthophène)s tels que le poly(3,4-dioctylthiophène), les poly(alcoxythiophènes), les polyfurannes, les polyazulènes, les polyazines, les polyanilines, et les poly(dioxythiophènes).

Avantageusement, ledit polymère conducteur de l'électricité C de type P est un polymère régiorégulier tel qu'un polyalkylthiophène régiorégulier par exemple le poly(3-octylthiophène).

Le polymère conducteur de l'électricité C pourra être notamment choisi parmi les polymères suivants : 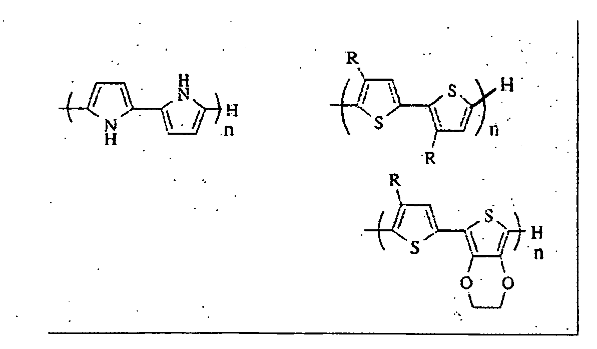 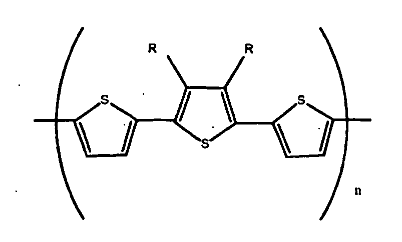 où n représente un nombre entier de 1 à 1 000, de préférence de 5 à 100, et R représente un groupe choisi parmi les groupes alkyle de 1 à 24C, de préférence de 4 à 12C, et les groupes alcoxy de 1 à 24C, de préférence de 4 à 12C.

Le groupe espaceur déconjuguant E est un élément structurel fondamental des complexes, colorants, selon l'invention.

Par espaceur, on entend généralement un motif constitué d'au moins un atome, séparant deux entités fonctionnelles.

Par déconjuguant, on entend généralement que le groupe réalise une rupture de la conjugaison par rupture du recouvrement des orbitales π.

Les groupes qui peuvent convenir pour de tels groupes espaceurs déconjuguants peuvent être facilement déterminés par l'homme du métier. Ainsi, ledit groupe espaceur déconjuguant E peut être choisi parmi les groupées : 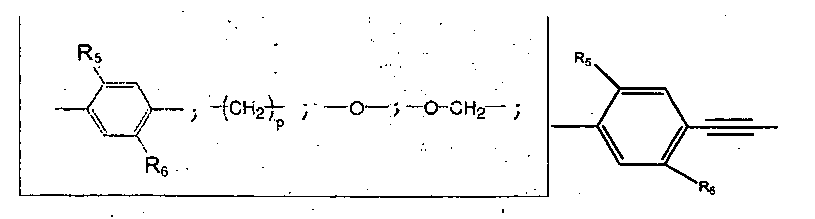 où R₅ et R₆ identiques ou différents sont généralement choisis, entre autres, parmi les groupes alkyles de 1 à 24C, de préférence de 1 à 12C et les groupes alcoxy de 1 à 24C, de préférence de 1 à 12C ; p est un nombre entier de 1 à 20, de préférence de 1 à 4.

Les complexes, colorants, préférés selon l'invention répondant à la formule (III) ou à la formule (IIIA) suivante : 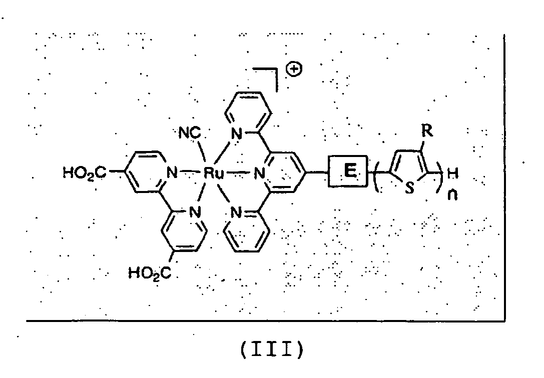 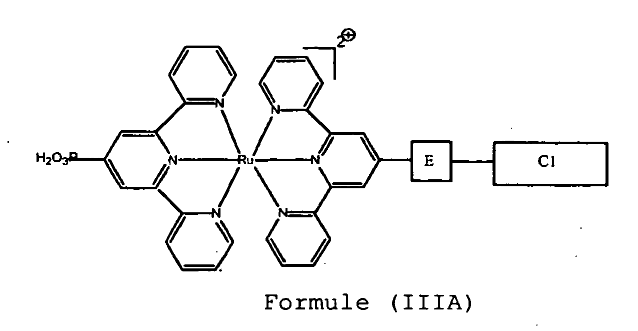 dans laquelle n représente un nombre entier de 1 à 1 000, de préférence de 5 à 100 ; R représente un groupe alkyle de 1 à 24C, de préférence de 4 à 12C, ou un groupe alcoxy de 1 à 24C, de préférence de 4 à 12C, et E est choisi parmi les groupes 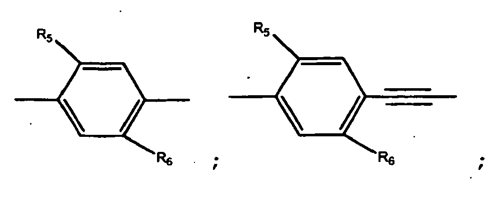 et -(CH₂)₂-,
R₅ et R₆ identiques ou différents sont choisis parmi les groupes alkyle de 1 à 24C, de préférence de 1 à 12C et les groupes alcoxy de 1 à 24C, de préférence de 1 à 12C ; et C1 représente : 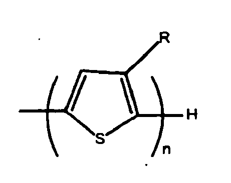 ou

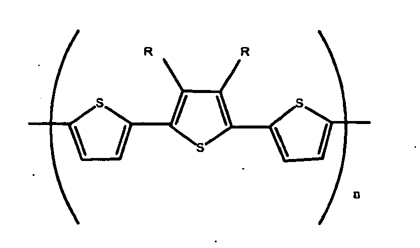

L'invention concerne en outre un procédé de préparation des composés préférés (III) tels que décrits ci-dessus, où E représente -(CH₂)₂- ou 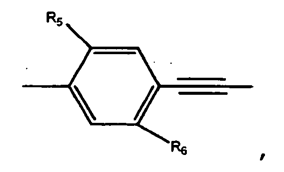 dans lequel on réalise les étapes successives suivantes :
a) - on fait réagir un composé de formule 1 suivante ou de formule 12 suivante : 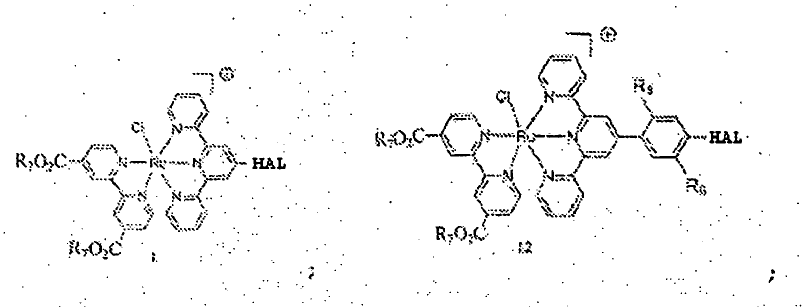 avec un composé de formule 2 suivante : 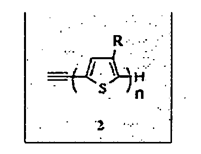 dans lesquelles n représente un nombre entier de 1 à 1 000, de préférence de 5 à 100 et R, R₅, R₆ et R₇ identiques ou différents représentent un groupe alkyle de 1 à 24C, de préférence de 4 à 12C pour R, et de préférence de 1 à 12C pour R₅, R₆ et R₇, ou un groupe alcoxy de 1 à 24C, de préférence de 4 à 12C pour R, et de préférence de 1 à 12C pour R₅, R₆ et R₇, de préférence encore R₇ est un groupe éthyle, et HAL représente un atome d'halogène, de préférence un atome de Br ; selon une réaction de SONOGASHIRA, dans un mélange de DMF/THF, en présence d'un système catalytique comprenant de l'iodure de cuivre, du [1,1'-bis(diphénylphosphino) ferrocène] dichloro-palladium (II) et de la triéthylamine, pour obtenir respectivement un composé de formule 3 suivante, ou un composé de formule 13 suivante : 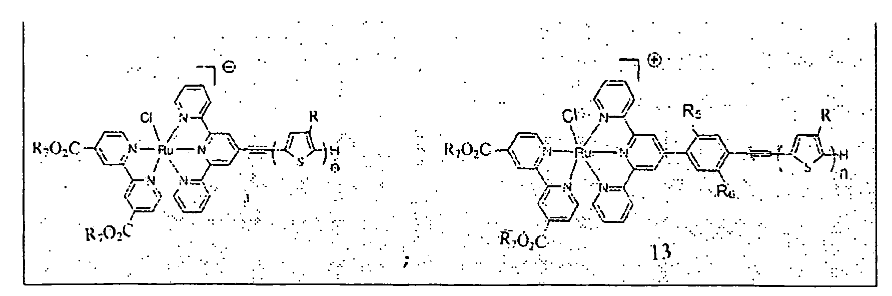
b) - on fait réagir le composé de formule 3 avec de l'hydrogène dans le THF en présence de palladium sur charbon pour obtenir un composé de formule 4 suivante : 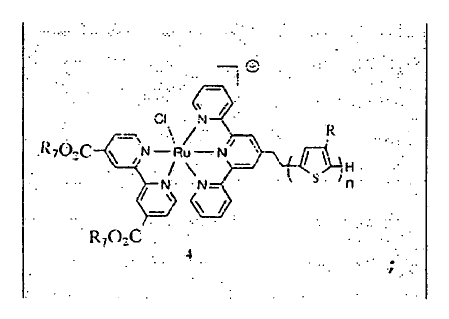
c) - on fait réagir le composé de formule 4 ou le composé de formule 13 dans un mélange de THF/H₂O, avec KCN/LiOH pour obtenir respectivement un composé de formule 5 suivante ou un composé de formule 14 suivante :

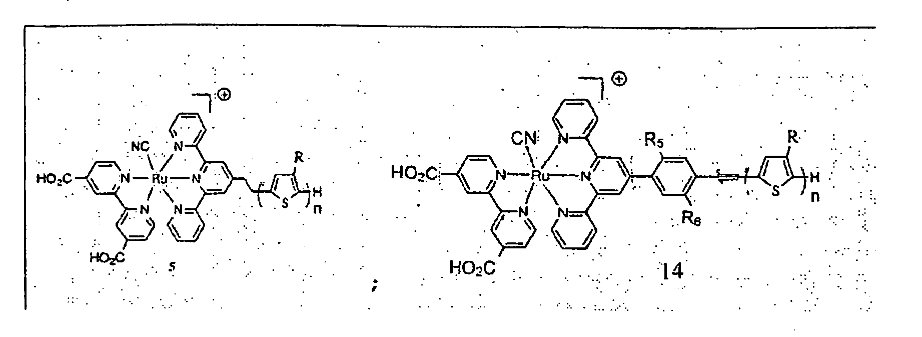

Il est à noter que ce procédé peut aussi être mis en oeuvre pour préparer les composés de formule (IIIA) moyennant quelques ajustements et adaptations à la portée de l'homme du métier.

Le composé de formule 12 ci-dessus peut être préparé par réaction d'un composé 10 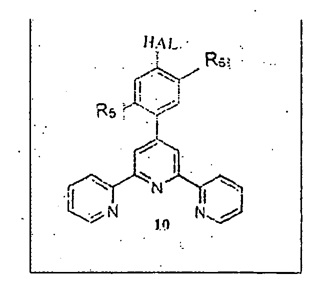 où HAL représente un atome d'halogène, de préférence un atome de Br
avec un composé 11

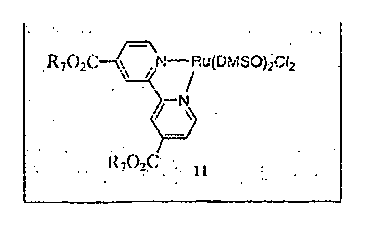

L'invention concerne en outre un composé 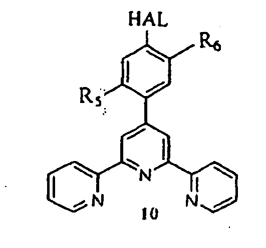 où HAL représente un atome d'halogène, de préférence un atome de Br,
qui est un composé intermédiaire nouveau.

Les schémas ci-dessous illustrent les voies de synthèse des composés de formule (III) :

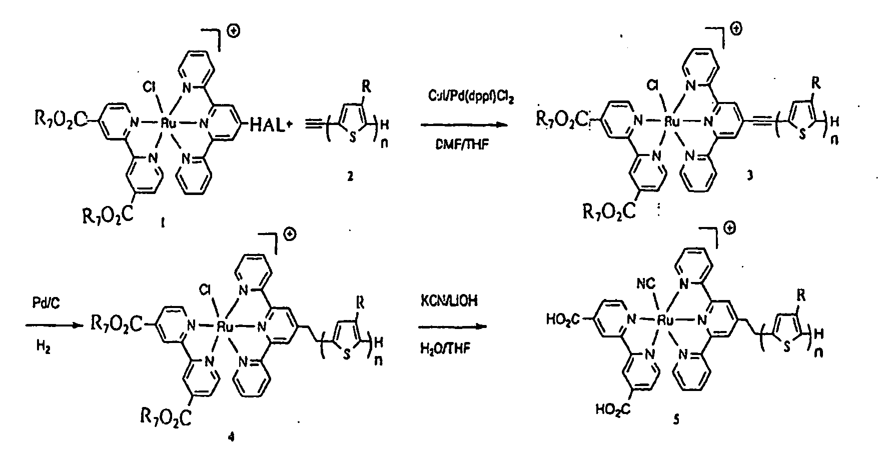

Schéma 1 : synthèse de la molécule 5 (R et R₇ représentent une chaîne alkyle ou alcoxy de 1 à 24C et de préférence de 4 à 12C pour R, ou de préférence de 1 à 12C pour R₇ ; R₇ est, de préférence encore Et).

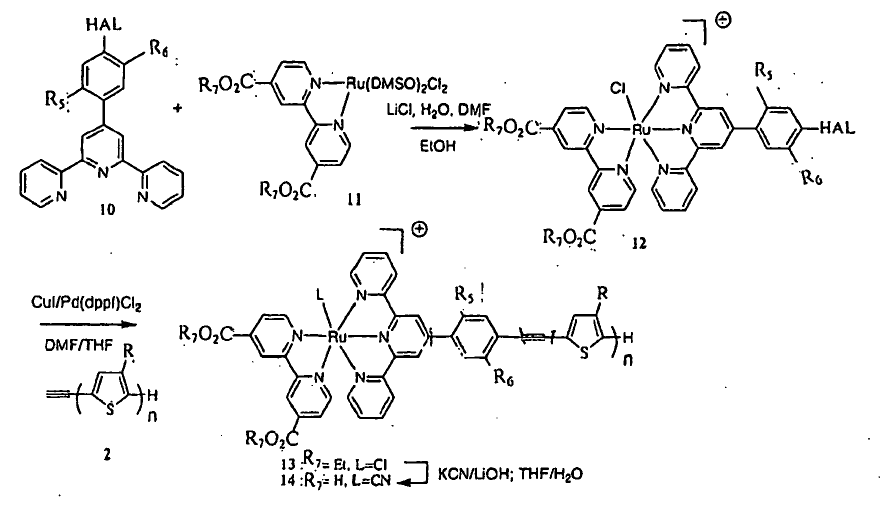

Schéma 2 : préparation du composé **14.** R, R₅, R₆, R₇ représentent une chaîne alkyle ou alcoxy de 1 à 24C, de préférence de 4 à 12C pour R, et de préférence de 1 à 12C pour R₅, R₆ et R₇ ; R₇ est, de préférence encore Et, et dans le composé 14, R₇, à titre d'exception, représente H.

Le complexe **1** est préparé selon un protocole de la littérature décrit par Odobel [29] et le polymère **2** est quant à lui obtenu selon une méthodologie publiée par McCullough [30]. Le greffage de 1 sur 2 se fait par une réaction de Sonogashira avec le catalyseur Pd(dppf)Cl₂ (dppf=diphénylephosphoferrocène).

La synthèse du complexe **14** est réalisée selon une série de réactions similaire à celle permettant de préparer 5 à partir du complexe **12** et du polymère **2.**

**12** est préparé à partir du complexe **11** décrit par Odobel [29] (schéma 2) et du ligand 10.

Le nouveau ligand **10** est préparé selon la séquence de réaction illustrée au schéma 3. La molécule **9** est synthétisée à partir des précurseurs commerciaux **6** et **7** par condensation de l'aldéhyde **8** et de l'acétyle pyridine **7.** La cyclisation de la pyridone **9** par l'acétate d'ammonium conduit à la terpyridine **10.**

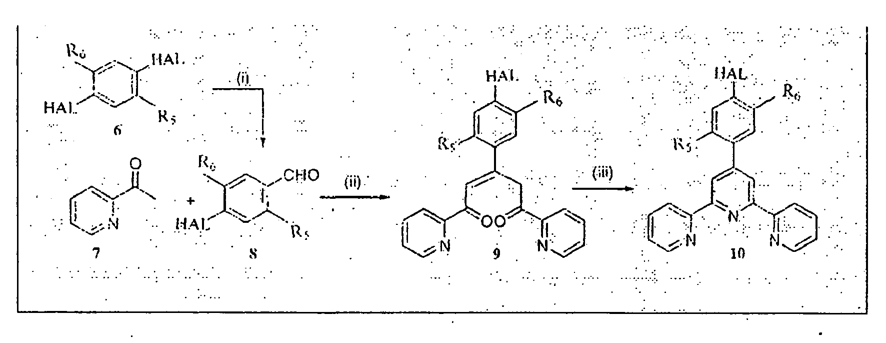

Schéma 3 : synthèse de la terpyridine **10** ; **conditions et réactifs :** i) 1. Buli, THF, 2. DMF ii) tBuOK, THF iii) NH₄OAc, EtOH. HAL représente de préférence Br.

Le complexe 12' peut également être facilement obtenu par hydrolyse du complexe 12. Ce complexe peut être utilisé sans greffage de polymère. Néanmoims, le greffage d'un polymère conducteur conformément à l'invention permet d'augmenter sensiblement les rendements photovoltaïques.

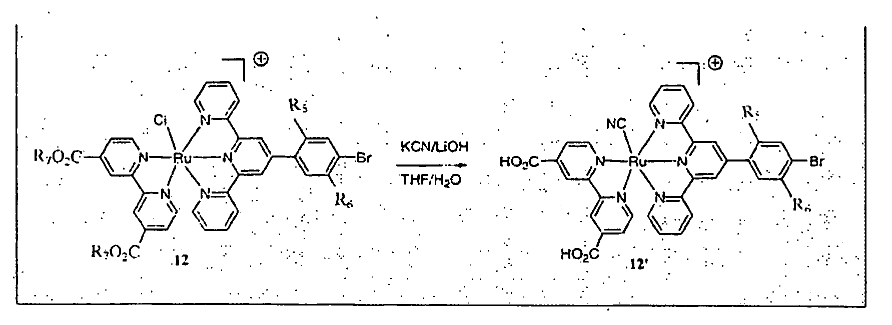

R₅, R₆, R₇ sont tels que définis plus haut. De préférence, R₇ est éthyle.

L'invention concerne aussi un matériau hybride inorganique-organique semiconducteur de type P-N comprenant une céramique oxyde poreuse semiconductrice à laquelle est greffée chimiquement un complexe, colorant, de formule (I) tel qu'il est défini, plus haut.

Ce matériau est nouveau et présente des propriétés avantageuses liées au complexe de formule (I) qui ont été déjà décrites plus haut.

Comme cela a été mentionné précédemment, le substrat est en céramique oxyde poreuse semi-conductrice. Il est entendu que, selon que le ou les polymères conducteurs de l'électricité sont des polymères de type N ou P, la céramique oxyde sera choisie de manière à être de type P ou N, ce choix étant à la portée de l'homme du métier. Les céramiques oxydes peuvent être des céramiques à base de métaux de transition choisis parmi Ti, V, Cr, Mn, Fe, Co, Ni, Cu, Zn, Y, Zr, Nb, Mo, Ru, Rh, Pd, Ag, Cd, Hf, Ta, W, Re, Os, Ir, Pt ou à base de lanthanides, tels que La, Ce, Pr, Nd, Sm, Eu, Gd, Tb, Dy , Er, et Yb ou à base d'éléments du groupe IIIA de la classification périodique choisis parmi Al, Ga, In et Tl ou à base d'éléments du groupe IVA de la classification périodique choisis parmi Si, Ge, Sn et Pb ou à base d'éléments du groupe VIA de la classification périodique choisis parmi Se et Te. Les céramiques oxydes peuvent être aussi toutes combinaisons entre métaux de transition, entre lanthanides, entre éléments du groupe IIIA, entre éléments du groupe IVA et éléments du groupe VIA.

Au sens de la présente invention, on entend par « céramique oxyde poreuse » une céramique métallique présentant des atomes d'oxygène et présentant une porosité globalement ouverte. Des céramiques appropriées peuvent être des céramiques oxydes amorphes, nanocristallines et/ou mésoporeuses.

Par céramique oxyde amorphe, on entend une céramique ne présentant pas de cristallites ou des cristallites de taille sub-nanométriques.

Par céramique oxyde nanocristalline, on entend une céramique présentant des cristallites de l'ordre de quelques nanomètres, par exemple de 2 à 200 nm.

Enfin, par céramique oxyde mésoporeuse, on entend une céramique se caractérisant par une importante porosité, avec des tailles de pores allant de 2 à 80 nm et des parois de 5 à 30 nanomètres d'épaisseur. Les pores sont généralement répartis de manière aléatoire avec une distribution très large de la taille des pores, dans la gamme mentionnée ci-dessus. Les céramiques mésoporeuses utilisées selon l'invention sont avantageusement des céramiques « mésostructurées », qui se présentent sous forme de réseaux poreux organisés qui présentent un agencement spatial ordonné de mésopores. Cette périodicité spatiale des pores est caractérisée par l'apparition d'au moins un pic à bas angle dans un diagramme de diffusion des rayons X ; ce pic est associé à une distance de répétition qui est généralement comprise entre 2 et 50 nm. Les matériaux mésostructurés se caractérisent par une maximisation de la surface par un volume donné et l'assurance de la continuité du réseau solide selon au moins une direction de l'espace au travers des parois constituant ledit matériau.

Un exemple de céramique oxyde poreuse pouvant être utilisée selon l'invention est du dioxyde de titane TiO₂. D'autres exemples selon le ZnO ou le SnO₂.

Parmi les céramiques envisagées dans le paragraphe précédent, l'homme de l'art peut choisir des céramiques de type N (auquel cas le polymère conducteur C greffé par l'intermédiaire du complexe selon l'invention sera un polymère de type P) et ou des céramiques de type P (auquel cas le polymère conducteur greffé par l'intermédiaire du complexe selon l'invention sera un polymère de type N)

Dans le matériau hybride, selon l'invention, la surface ainsi que l'intérieur du substrat en céramique poreuse est greffé, par un ou plusieurs complexes définis plus haut qui comportent un polymère conducteur de l'électricité tel que défini ci-dessus.

L'invention a également trait à un procédé de préparation du matériau hybride inorganique-organique semiconducteur tel que défini précédemment dans lequel on imprègne une céramique oxyde poreuse semiconductrice par une solution organique comprenant un ou plusieurs complexes de formule (I) tels que définis plus haut.

Comme il est précisé plus haut, dans le matériau hybride selon l'invention, une surface en céramique oxyde poreuse est greffée par des composés de formule (I) tel que définis précédemment, lesdits composés se greffant chimiquement à ladite surface de la céramique.

Pour obtenir un tel greffage, différentes techniques peuvent être envisagées, en particulier les techniques par voie liquide, c'est-à-dire les techniques d'imprégnation du substrat en céramique oxyde poreuse semiconductrice sus-mentionné avec une solution organique comprenant le ou les composés de formule (I) tels que définis ci-dessus.

Ainsi, le greffage chimique à la surface et à l'intérieur de la céramique oxyde poreuse peut s'effectuer par l'une des techniques suivantes :
- le trempage-retrait (connu sous la terminologie anglaise « dip-coating ») ;
- l'enduction centrifuge (connu sous la terminologie anglaise « spin-coating ») ;
- l'enduction laminaire (connu sous la terminologie anglaise « laminar-flow-coating ») ;
- la pulvérisation (connu sous la terminologie anglaise « spray-coating »)
- l'épandage (connu sous la terminologie anglaise « soak coating ») ;
- l'enduction au rouleau (connu sous la terminologie « roll to roll process ») ;
- l'enduction au pinceau (connu sous la terminologie anglaise « painting coating ») ;
- la sérigraphie (connu sous la terminologie anglaise « screen printing »).

Ces différentes techniques doivent être mises en oeuvre pendant une durée appropriée, de manière à permettre un contact optimum du substrat en céramique oxyde poreuse avec la solution organique comprenant le ou les composés (I) aptes à être greffés, de façon à ce que le substrat soit imprégné à la fois à sa surface et en son intérieur et à ce que les composés puissent réagir et se lier chimiquement à la surface et à l'intérieur dudit substrat. Par exemple, cette durée pourra être de 1 à 48 heures, par exemple de 16 heures.

Le solvant de ladite solution peut être facilement choisi par l'homme du métier.

Ce solvant pourra ainsi être choisi parmi le THF, les alcools aliphatiques de 1 à 4C tels que le méthanol et l'éthanol, les solvants halogénés, et leurs mélanges.

La concentration du complexe, colorant (I), dans ladite solution peut être facilement déterminée par l'homme du métier, elle est généralement de 10⁻³ à 1 M.

La température à laquelle est réalisée l'imprégnation peut être, de même, facilement déterminée par l'homme du métier, elle est généralement de 20 à 80°C, de préférence cette imprégnation est réalisée à la température ambiante.

Après cette étape de greffage ou fonctionnalisation, le procédé de préparation du matériau hybride inorganique-organique, l'invention peut comprendre une étape de traitement destiné à éliminer les résidus de la réaction de greffage ainsi que les espèces n'ayant pas réagi.

Ce traitement peut consister en un rinçage du matériau hybride par un solvant aqueux ou organique qui est de préférence le même solvant que celui utilisé pour le greffage.

Enfin, on procède généralement à un séchage du matériau hybride inorganique-organique.

Les matériaux hybrides inorganiques-organiques semiconducteurs de l'invention peuvent être utilisés dans différents dispositifs nécessitant la présence d'un matériau semi-conducteur tels que des dispositifs électrochimiques, des dispositifs photo-électrochimiques et des dispositifs de catalyse et en particulier dans des cellules photovoltaïques ou les diodes électroluminescentes.

Ainsi, la présente invention a également pour objet une cellule photovoltaïque comprenant :
- une première électrode collectrice de courant (dite « électrode de travail ») ;
- une seconde électrode (dite « contre-électrode de travail ») ;
- une zone semi-conductrice constituée du matériau hybride semiconducteur tel que défini précédemment, ladite zone étant disposée entre ladite première électrode et ladite seconde électrode.

La première électrode, ou électrode de travail comprend une partie conductrice sous forme, par exemple, d'une couche d'oxyde d'étain dopé au fluor, ou d'ITO, cette partie pouvant être déposée sur un support.

On précise que par support, on entend, au sens de l'invention, tout substrat, organique ou inorganique, se caractérisant par une transparence au moins égale à 50% dans le spectre solaire. Ce support peut être par exemple en verre transparent.

Il est noté que la partie conductrice susmentionnée sera en contact avec la zone semi-conductrice susmentionnée, soit de manière directe, soit par le biais par exemple d'une couche de dioxyde de titane dense, cette dernière permettant d'éviter un contact direct entre l'électrode de travail et la zone semi-conductrice et de ce fait un court-circuit dans la cellule photovoltaïque.

Il est précisé également qu'une couche à base de polymère conducteur de l'électricité ou d'une autre molécule conductrice de type P peut être interposée entre ladite zone semi-conductrice en matériau hybride semiconducteur selon l'invention et la seconde électrode (dite « contre-électrode de travail »), de manière à éviter un court-circuit dans la cellule photovoltaïque.

En effet, généralement, le complexe « FSEC » de formule (I) est autosuffisant, c'est-à-dire que la longueur de la chaîne du polymère C est suffisante pour sortir des pores du matériau céramique oxyde poreux et assurer le contact avec la seconde électrode. Auquel cas ladite couche de polymère conducteur de l'électricité n'est pas nécessaire.

Il est cependant probable que dans certains cas la chaîne C ne soit pas assez longue auquel cas le matériau hybride actif est recouvert d'un matériau solide conducteur de type P (ou N) constitué généralement par un polymère conducteur de l'électricité tel qu'un poly(alkylthiophène) régiorégulier comme le poly(3-octylthiophène) ou par une autre molécule conductrice de type P telle qu'une amine aromatique.

De préférence, on choisit pour cette couche supplémentaire un polymère conducteur de l'électricité identique au polymère conducteur de l'électricité C du complexe (I) faisant partie du matériau hybride semiconducteur, tel qu'un poly(3-octylthiophène).

Ladite couche supplémentaire optionnelle de polymère semiconducteur de l'électricité peut être préparée par toute technique adéquate par exemple par une technique par voie humide telle que déjà décrite plus haut, par exemple une technique mettant en oeuvre une solution du polymère dans un solvant, telle que l'enduction centrifuge.

Généralement, la seconde électrode (ou « contre-électrode de travail ») se présente sous la forme d'une couche métallique, par exemple une couche métallique à base d'or et/ou de nickel.

Les cellules photovoltaïques conçues à partir du matériau hybride inorganique-organique semi-conducteur P-N de l'invention présentent notamment les avantages suivants par rapport aux cellules existantes :
1 - un rendement de photo conversion plus important notamment en ce qui concerne la densité de photocourant, le photo-potentiel et le facteur de forme.
2 - L'absence de problèmes liés à la présence d'un électrolyte liquide, c'est-à-dire que le scellement hermétique des deux électrodes de la cellule ne se pose plus.
3 - Comptabilité de ce type de cellules avec la fabrication de dispositifs sur substrat flexible (plastique par exemple).
4 - Possibilité d'utiliser les cellules en éclairage extérieur en présence d'humidité atmosphérique et où la température peut atteindre 50-60°C.
5 - Possibilité de développement industriel car tous les constituants de la cellule sont des composants de faible coût et compatibles avec les techniques de dépôt en continu (ex. roll to roll).
6 - Possiblité d'utiliser des fils de conducteurs électriques métalliques immergés dans le verre conducteur pour drainer le courant dans le circuit électrique externe.

Les avantages de ces cellules sont notamment liés à la mise en oeuvre du matériau hybride tel que décrit plus haut, et à l'utilisation du complexe (I) tel que décrit ci-dessus.

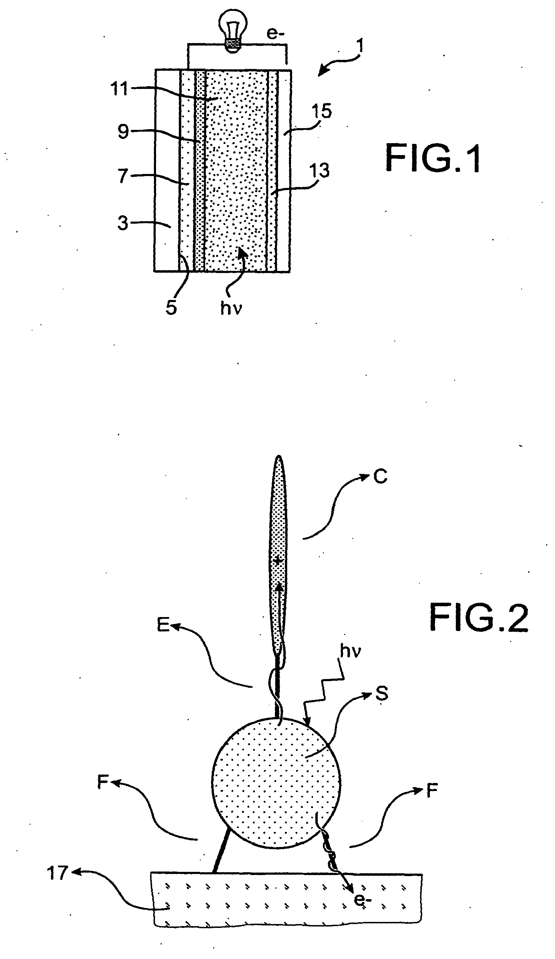

En se référant à la figure 1, on voit une cellule photovoltaïque conforme à la présente invention désignée par la référence générale 1.

La cellule 1 comprend un support 3 transparent en verre recouvert sur une face 5 par une couche conductrice transparente 7, cette couche pouvant être à base d'oxyde d'étain dopé au fluor ou d'ITO. Le support revêtu de la couche conductrice transparente fait office d'électrode collectrice de courant (dite première électrode selon la terminologie employée ci-dessus).

Une couche dense de dioxyde de titane 9 est déposée sur la couche conductrice transparente 7. Sur cette couche dense est disposée une couche de matériau semi-conducteur 11, ledit matériau semi-conducteur correspondant au matériau hybride inorganique-organique semi-conducteur P-N de l'invention. Sur cette couche en matériau semi-conducteur 11 est déposée une couche en polymère conducteur 13 (cette couche peut être éventuellement omise), sur laquelle est déposée une couche métallique 15, par exemple une couche à base d'or et/ou de nickel et/ou d'argent et/ou d'aluminium. La couche éventuelle en polymère conducteur 13, prise en sandwich entre la couche en matériau semi-conducteur 11 et la couche métallique 15, permet de limiter les phénomènes de court circuit. La couche métallique 15 fait office de contre-électrode (dite seconde électrode selon la terminologie employée ci-dessus).

La figure 2 représente une partie agrandie de la couche en matériau semi-conducteur 11 et montre plus précisément l'interface entre la surface du substrat en céramique oxyde poreuse et le complexe de formule (I) selon l'invention : FSEC.

Sur cette figure, la référence 17 désigne une surface de la paroi d'un pore de la céramique oxyde poreuse semiconductrice.

La surface 17 est sensibilisée par greffage des composés de formule (I) selon l'invention. Lorsqu'un rayon lumineux atteint la substance chromophore (ledit rayon lumineux étant représentée par une flèche hv), l'énergie lumineuse qu'il transporte sous forme de photons est absorbée par la substance chromophore. Celle-ci se libère d'un électron e⁻ qui est directement, dans ce cas de figure, capté par la céramique oxyde poreuse, tandis que le trou de charge symbolisé + créé de manière concomittante à l'électron est capté par le polymère conducteur. Il y a ainsi une dissociation de la paire électron-trou sans recombinaison et donc création d'un courant électrique au sein de matériau.

La connexion, selon l'invention, par une liaison covalente, entre le sensibilisateur et la céramique d'une part, et avec le polymère conducteur d'autre part, permet une meilleure injection des charges dans les matériaux semiconducteurs (céramique et polymère).

Les cellules photovoltaïques de la présente invention peuvent être préparées de la façon suivante :
- une étape de dépôt sur un support recouvert éventuellement d'une couche conductrice transparente d'un film de céramique oxyde, ledit dépôt pouvant se faire par des techniques sous vide ou par voies humides précédemment décrites, ces deux types de procédés étant à la portée de l'homme du métier ;
- la mise en oeuvre du procédé de préparation du matériau semi-coriducteur PN tel que défini précédemment, de manière à obtenir à partir du film de céramique oxyde susmentionné ledit matériau semi-conducteur;
- éventuellement, une étape de dépôt sur la couche en matériau semiconducteur d'une couche en polymère conducteur de l'électricité, de préférence, identique à celui constitutif du matériau hybride inorganique-organique semi-conducteur P-N de l'invention, ladite couche étant déposée par des techniques par voie humide précédemment décrites, à la portée de l'homme du métier ;
- une étape de dépôt d'une couche métallique telle que définie précédemment sur la couche en matériau semi-conducteur ou le cas échéant sur la couche en polymère conducteur de l'électricité.

La présente invention va maintenant être décrite par rapport à un exemple de réalisation.

### BRÈVE DESCRIPTION DES DESSINS

La figure 1 correspond à une vue en coupe d'une cellule photovoltaïque de l'invention, déjà décrite.
La figure 2 correspond à une partie agrandie d'une partie de la cellule representée sur la figure 1, cette partie étant décrite ci-dessus.

L'invention va maintenant être décrite en référence aux exemples ci-dessous, donnés à titre illustratif et non limitatif.

### EXPOSÉ DÉTAILLÉ DE MODES DE RÉALISATION PARTICULIERS

### Exemple 1 : synthèse du complexe 5 (aussi appelé C13)

50 mg (0,06 mmol, 1 éq.) de complexe **1** tel que décrit plus haut dissous dans 2 mL de DMF distillé et 600 mg (0,12 mmol, 2 éq.) de polymère **2** tel que décrit plus haut et (où R est un groupe n-octyle et n=40) dissous dans 10 mL de THF distillé sont introduits dans un tube scellé. Après 3 dégazages sous argon, 9,5 mg (0,013 mmol, 0,2 éq.) de [1,1'-bis(diphénylphsophino)ferrocène] dichloro-palladium (II), 2,5 mg (0,013 mmol, 0,2 éq.) de iodure de cuivre et 0,5 mL de triéthylamine sont ajoutés. Le mélange est chauffé à 90°C pendant 20 heures. Après refroidissement du milieu, le solvant est évaporé sous pression réduite. Le produit obtenu est purifié par 2 chromatographies successives sur alumine (colonne 1 éluant : gradient méthanol dans le chloroforme : de 0 à 5%, colonne 2 éluant : gradient méthanol dans le chloroforme : de 0 à 1%) pour donner un solide violet noir (70 mg, 20%).
**RMN¹H** : (300 MHz dans CDCl₃, δppm)
10,56 (d, J³=6 Hz, 1H), 9,06 (s, 1H), 8,78 (s, 1H), 8,39 (s, 2H), 8,29 (d, J³=7,5 Hz, 2H), 7,86 (m, 3H), 7,72 (d, J³=5,4 Hz, 1H), 7,51 (d, J³=5,7 Hz, 2H), 7,27-7,16 (m, 3H), 6,98 (s, 38H), 4,63 et 4,39 (2q, 4H), 2,89 (m, 76H), 1,71-1,31 (m, 462H), 0,93 (t, 114H).
**UV-Visible** (THF, λₘₐₓen nm) : 265, 303, 445, 552.

### Synthèse de l'intermédiaire 4

130 mg (0,02 mmol, 1 éq.) de complexe 3 tel que décrit plus haut, où R est un groupe n-octyle et n=40, dissous dans 12 mL de THF distillé sont introduits dans un tricol. Après un dégazage sous argon, 20 mg (0,06 mmol, 3 éq.) de palladium sur charbon à 20% sont ajoutés avant l'introduction du dihydrogène. Le milieu réactionnel est agité pendant 14 heures à température ambiante. Le solvant est évaporé sous pression réduite et le produit bruit est purifié sur sephadex^{®} pour donner un solide violet noir (130 mg, quantitatif).
**RMN¹H** : (300 MHz dans CDCl₃, δppm)
6,98 (s, 38H), 2,89 (m, 76H), 1,71-1,31 (m, 456H), 0,93 (t, 114H).
**UV-Visible** (THF, λₘₐₓen nm) : 265, 303, 445

### Synthèse du complexe 5

150 mg (0,02 mmol, 1 éq.) de complexe 4 tel que décrit plus haut où R est un groupe n-octyle et n=40 dissous dans 10 mL de THF, 3 mL d'une solution de LiOH 0,2M et 20 mg (0,6 mmol, 30 éq.) de KCN sont introduits dans un ballon. Après 6 heures de chauffage à 90°C, le solvant est évaporé sous pression réduite. Le produit obtenu est purifié sur sephadex pour donner un solide violet noir (150 mg, quantitatif) qui est le complexe 5 dont la formule a déjà été donnée plus haut, où R est un groupe n-octyle et n=40.
**RMN¹H** : (300 MHz dans CDCl₃, δppm)
6,98 (s, 38H), 2,89 (m, 76H), 1,71-1,31 (m, 456H), 0,93 (t, 114H).
**UV-Visible** (THF, λₘₐₓen nm) : 265, 303, 445

### Exemple 2 : synthèse du complexe 12'

Synthèse de l'intermédiaire 4-brome-2,5-diméthylbenzaldéhyde **8 :**
Dans un tube de schlenck sous argon, 4 g (15 mmol., 1 éq.) de 1,4-dibromo-2,5-diméthylbenzène sont solubilisés dans 50 mL de THF distillé. Le milieu est alors refroidi à -78°C puis 6,6 mL (16,6 mmol., 1,1 éq.) de BuLi (2,5M dans l'hexane) sont ajoutés goutte à goutte. Après 1 heure d'agitation à cette température, 5,3 mL (68 mmol., 4,5 éq.) de DMF fraîchement distillé sont ajoutés. Le milieu réactionnel est de nouveau agité à cette température pendant 1 heure, puis remonte jusqu'à la température ambiante pendant la nuit. Ensuite, le milieu est dilué avec une solution de chlorure d'ammonium puis extrait 3 fois à l'acétate d'éthyle. Les phases organiques sont réunies, séchées sur sulfate de magnésium, et concentrées sous pression réduite. Le résidu est purifié par chromatographie sur colonne de silice (éluant : acétate d'éthyle/éther de pétrole : 80/20) pour donner un solide blanc (m : 1,3 g, Rdt : 40%).
   **RMN¹H** : (300 MHz dans CDCl₃, δppm)
   10,20 (s, 1H), 7,62 (s, 1H), 7,46 (s, 1H), 2,62 (s, 3H), 2,42 (s, 3H).

### Synthèse de l'intermédiaire 4'-(1-bromo-2,5-diméthyl-4-phényl) 2,2',6',2'' terpyridine 10 :

Dans un tricol, 0,57 g (4,7 mmol., 2 éq.) d'acétylpyridine sont additionnés à une suspension de 0,8 g (7 mmol. , 3 éq.) de tert-butanolate de potassium dans 30 mL de THF distillé. Après 30 minutes sous agitation à température ambiante, 0,53 g (2,3 mmol., 1 éq.) dé 4-bromo-2,5-diméthylbenzaldéhyde sont ajoutés goutte à goutte dans 5 mL de THF distillé. Après 20 heures d'agitation sous argon, 1,8 g (23 mmol, 10 éq.) d'acétate d'ammonium en solution dans un mélange d'éthanol (10 mL) et d'acide acétique (5 mL) sont ajoutés. Le milieu réactionnel est alors chauffé à 80°C pendant 6 heures. Le mélange est refroidi et versé sur un mélange glace+eau (100 g). Le précipité formé est filtré et lavé à l'éther diéthylique. La phase aqueuse est extraite 3 fois au dichlorométhane. Les phases organiques sont réunies, séchées sur sulfate de magnésium et concentrées sous pression réduite. Le résidu et le précipité sont réunis et purifiés par chromatographie sur colonne d'alumine (dépôt solide, éluant : gradient d'acétate d'éthyle dans l'éther de pétrole : de 0 à 30%) pour donner un solide blanc (m : 350 mg, Rdt : 36%).
**RMN¹H** : (300 MHz dans CDCl₃, δppm)
8,70 (d, J³=3,9 Hz, 2H), 8,67 (d, J³=7,8 Hz, 2H), 7,88 (dd, J⁴ = 1,2 Hz, J³=7,8 Hz, J³=7,8 Hz, 2H), 7,48 (s, 1H), 7,34 (ddd, J⁴=1 Hz, J³=4,8 Hz, J³=7,8 Hz, 2H) 7,24 (s, 1H), 2,40 (s, 3H), 2,31 (s, 3H).

### Synthèse de l'intermédiaire 12 :

60 mg (0,14 mmol, 1 éq.) de 4'-(1-bromo-2,5-diméthyl-4-phényl)-2,2',6',2'' terpyridine solubilisé dans un minimum de THF, 159 mg (0,26 mmol, 1,8 éq.) de dichloro bis diméthylsulfoxyde (4,4'-dicarboxylate de diéthyl-2,2'-bipyridine) ruthénium (II) solubilisé dans 8 mL d'éthanol et 30 mg (0,72 mmol, 5 éq.) de chlorure de lithium dissous dans 0,5 mL d'H₂O sont introduits dans un ballon de 25 mL. Le mélange est chauffé au reflux de l'éthanol pendant 16 heures. Après refroidissement du milieu, le solvant est évaporé sous pression réduite. Le produit obtenu est solubilisé dans un minimum d'acétone puis l'ajout d'une solution saturé en tétrafluoroborate de sodium entraîne la précipitation d'un solide rose isolé par filtration. Le produit obtenu est de nouveau solubilisé dans un minimum dichlorométhane et d'acétone puis précipité par ajout d'éther diéthylique pour donner un solide rose (61 mg, 45%).
**RMN¹H** : (300 MHz dans CDCl₃, δppm)
10,6 (d, J³=5,7 Hz, 2H), 9,04 (s, 2H), 8,74 (s, 2H), 8,37 (d, J³=4,8 Hz, H), 8,31 (s, 2H), 8,26 (d, J³=8,1 Hz, 2H), 7,92 (d, J³=5,7 Hz, 2H), 7,83 (m, 3H), 7,61 (s, 1H), 7,48 (m, 3H), 7,17 (t, J³ et J³=5,8 Hz, 2H), 4,64 (q, 2H), 4,35 (q, 2H), 2,46 et 2,44 (2s, 6H), 1,51 (t, 3H), 1,31 (t, 3H).

### Synthèse du complexe 12' :

Dans un ballon de 25 mL, 1 mL d'une solution d'hydroxyde de lithium à 4M est ajouté à 9 mg de complexe 12 et 3 mg de cyanure de potassium dissous dans 3 mL de DMF. Le milieu réactionnel est chauffé à 120°C pendant 16 heures. Après retour à température ambiante, l'ajout d'éther diéthylique entraîne la précipitation d'un solide orange. Le produit est filtré, solubilisé dans un minimum de méthanol puis reprécipité par ajout de quelques gouttes de HBF₄ puis d'eau. Le produit est purifié sur Sephadex^{®} pour donner un solide orange (m : 7,7 mg, Rdt : 86%).
**RMN¹H** : (300 MHz dans MeOD, δppm)
10,3 (d, J³=5,7 Hz, 2H), 9,04 (s, 2H), 8,74 (s, 2H), 8, 7 (d, J³=4,8 Hz, 1H), 8,31 (s, 2H), 8,26 (d, J³=8,1 Hz, 2H), 7,92 (d, J³=5,7 Hz, 2H), 7,83 (m, 3H), 7,61 (s, 1H), 7,84 (m, 3H), 7,17 (t, J³ et J³=5,8 Hz, 2H), 2,46 et 2,44 (2s, 6H).
**UV-Visible** (THF, λₘₐₓen nm) : 283, 305, 495

### Exempte 3 : Synthèse du complexe C3 de formule suivante :

Acide 4'-phosphonique-2,2':6',2''-terpyridine-(4'-[(3',4'-dioctyl-2,2':5',2''-terthiophèn)-5-yl]-2,2':6',2''-terpyridine) ruthénium (II) di-tétrafluoroborate

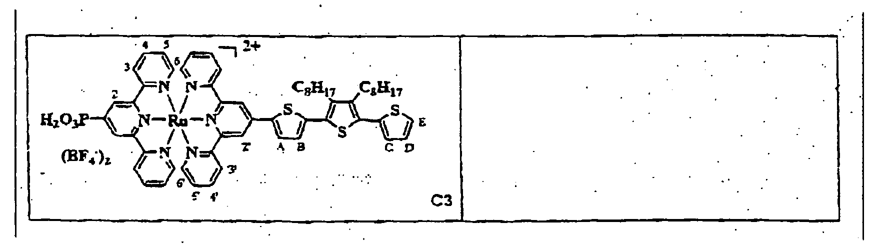

Synthèse du complexe intermédiaire C3a : 4'-[(3',4'-dioctyl-2,2':5',2''-terthiophène)-5-yl]-2,2':6',2''-terpyridine

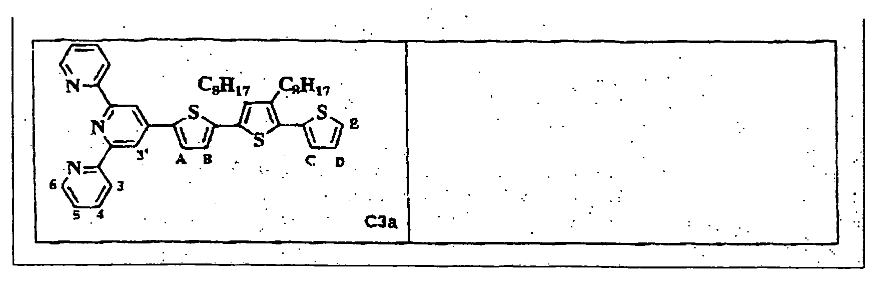

100 mg (0,25 mmol, 1 éq.) de 4'-triméthylstannyl-2,2' :6',2''-terpyridine, 200 mg (0,38 mmol, 1,2 éq.) de 5-bromo-3',4'-dioctyl-2,2' : 5',2''-therthiophène et 15 mL de dioxane distillé sont introduits dans un tube scellé. Après 3 dégazages sous l'argon, 26 mg (0,025 mmol, 0,045 éq.) de tris(dibenzylidèneacétone)dipalladium, 85 mg (0,55 mmol, 2,2 éq.) de fluorure de césium et 18 mg (0,09 mmol, 0,18 éq.) de tri-tert-butylphosphine sont ajoutés. Le mélange est chauffé à 130°C pendant 16 heures. Après refroidissement du milieu, la solution est diluée avec de l'acétate d'éthyle, lavé avec une solution dilué d'ammoniac et à l'eau. La phase organique est séchée sur sulfate de magnésium, filtrée puis concentrée sous vide. Le produit obtenu est purifié par chromatographie sur silice (éluant : gradient d'éther diéthylique dans le dichlorométhane (de 0 à 40%) pour donner un solide orange (143 mg, 80%).
**RMN ¹H :** (300 MHz dans CDCl₃, δppm)
8,74 (d, ³J₆₋₅=4,8 Hz, 2H, H₅), 8,68 (s, 2H, H_{3'}), 8, 62 (d, ³J₃₋₄=7,8 Hz, 2H, H₃), 7,88 (ddd, ³J₄₋₃=7,8 Hz, ³J₄₋₅=7,8 Hz, ⁴J₄₋₆=1,8 Hz, 2H, H₄), 7,74 (d, ³J_{A-B}=3,9 Hz, 1H, H_{A}), 7,37 (ddd, ³J₅₋₄=7,8 Hz, ³J₅₋₆=4,8 Hz, ⁴J₅₋₃= 1,8 Hz, 2H, H₅), 7.33 (dd, ³J_{E-D}=5,1 Hz, ³J_{D-C}=3,6, ³J_{E-C}= 1,2 Hz, 1H, H_{E}), 7,19 (d, ³J_{B-A}=3,9 Hz, 1H, H_{B}), 7,17 (dd, ³J_{C-D}=3, 6 Hz, ³J_{C-E}=1,2 Hz, 1H, H_{C}), 7,07 (dd, ³J_{D-E}= 5,1 Hz, 1H, H_{D}), 2,78 (t, 4H CH₂ alk en α), 2,71 (t, 4H, CH₂ alk en β), 1,28-1,62 (m, 30H, CH₂ alk), 0,87 (m, 6H, CH₃ alk).
**RMN ¹³ C** : (300 MHz dans CDCl₃, δppm)
14,1 ; 22,6 ; 28,1 ; 29,3 ; 29,9 ; 30,6 ; 30,7 ; 31,9 ; 116,7 ; 121,3 ; 123,8 ; 125,4 ; 125,9 ; 126, 2 ; 126,6 ; 127,4 ; 129,5 ; 130,4 ; 136,1 ; 136,8 ; 138,2 ; 140,3 ; 140,7 ; 140,9 ; 143,1 ; 199,1 ; 156,0.

Synthèse du complexe intermédiaire C3b :
4'-diéthylphosphonate-2,2':6',2 "-terpyridine-(4'-[(3',4'-dioctyl-2,2':5',2''-terthiophèn)-5-yl]-2,2':6',2''-terpyridine ruthénium (II) di-tétrafluoroborate (17) 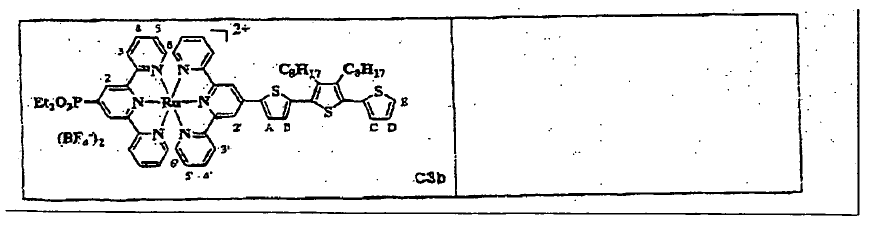
0,16 mmol, 1,1 éq. de trichlorure de 4'-diéthylphosphonate-2,2' :6',2''-terpyridine ruthénium (III) et 91 mg (0,47 mmol, 3,3 éq.) de tétrafluoroborate d'argent sont chauffés à 60°C pendant 1h30 dans un mélange dégazé d'éthanol (20 mL) et de DMF distillé (5 mL). Après refroidissement du milieu, la solution est filtrée afin d'éliminer le chlorure d'argent formé. Le filtrat est dégazé et de nouveau introduit en réaction avec une solution de 91 mg (0,14 mmol, 1 éq.) de 4'-[(3',4'-dioctyl-2,2' :5,2''-terthiophèn)-5-yl]-2,2' :6,2''-terpyridine et 60 mg (0,56 mmol, 4 éq.) (C3a) d'hydroquinone dans 4 mL de DMF distillé. Le milieu réactionnel est chauffé à reflux pendant 5 heures. L'ajout d'éther diéthylique entraîne la précipitation d'un solide qui est filtré, lavé à l'éther diéthyliqué et séché sous vide. Le produit obtenu est purifié par chromatographie sur silice (éluant : graduent d'eau et d'une solution de KNO₃ dans l'acétone (de 90:10:0 à 70:20:1 acétone/H₂O/KNO₃) pour donner un solide rouge. Le complexe est dissous dans un minimum de méthanol et précipité par ajout d'une solution saturée de NaBF₄ pour donner un solide rouge (105 mg, 60%).
   **RMN ¹H :** (300 MHz dans CDCl₃ αppm)
   8,90 (d, 2H, ³J_{2-P}=13 Hz, H₂), 8,86 (s, 2H, H_{2'}), 8,60 et 8,47 (2d, 4H, ³J₃₋₄ et ³J_{3'-4'}=7,8 Hz, H₃ et H_{3'}), 8,16 (d, 1H, ³J_{A-B}=3,3 Hz, H_{A}), 7,82 (m, 4H, H₄ et H_{4'}), 7,49 et 7,32 (2d, 4H, ³J₆₋₅) et ³J_{6'-5'}=5,2 Hz, H₆ et H_{6'}) 7,41 (dd, 1H. ³J_{E-D}=4,8 Hz, ⁴J_{E-C}=1,5 Hz, H_{E}), 7,24 (m, 2H, H_{B} et H_{C}), 7,14 (m, 4H, H₅ et H_{5'}), 6,81 (m, 1H, H_{D}), 4,48 (q, 4H, OCH₂CH₃), 2,77 (t, 4H CH₂ alk en α), 2,68 (t, 4H, CH₂ alk en β), 1,25-1,67 (m, 30H, OCH₂CH₃ et CH₂ alk), 0,88 (m, 6H, CH₃ alk).
   **RMN ¹³C** : (300 MHz dans CDCl₃, δppm)
   14,08 ; 16,52 ; 22,67 ; 29,26 ; 29,31 ; 29,68 ; 29,91 ; 29,98 ; 30,84 ; 31,90 ; 64,24 ; 119,16 ; 119,50 ; 124,95 ; 125,23 ; 127,51 ; 127,69 ; 128,01 ; 128,15 ; 128,25 ; 128,58 ; 129,80 ; 130,84 ; 131,31 ; 133,67 ; 137,84 ; 138,08 ; 138,36 ; 140,55 ; 140,68 ; 140,88 ; 141,67 ; 145,89 ; 151,91 ; 152,64 ; 154,65 ; 155,39 ; 157,45 ; 157,51.

Synthèse finale du complexe C3 :
Dans un tube scellé, 70 mg (0,05 mmol, 1 éq.) de complexe C3b sont introduits dans 10 mL de DMF fraîchement distillé et 400 mg (2,5 mmol, 35 éq.) de bromotriméthylsilane anhydre sont alors précautionneusement ajoutés sous argon. Le milieu réactionnel est chauffé à 50°C pendant 36 heures sous argon. Après retour à température ambiante, l'ajout de dichlorométhane entraîne la précipitation d'un solide qui est filtré, lavé au dichlorométhane et séché sous vide. Le complexe est dissous dans un minimum de méthanol et précipité par ajout d'une solution saturée de NaBF₄ pour donner un solide rouge purifié sur colonne Sephadex (65 mg, 90%).
   **RMN ¹H :** (300 MHz dans CDCl₃/MeOD (90/10), δppm)
   9,10 (d, 2H, ³J₂₋ₚ= 13 Hz, H₂), 8,81 (s, 2H, H_{2'}), 8,58 (d, 4H, ³J₃₋₄ et ³J_{3'-4'} =8,4 Hz, H₃ et H_{3'}, 8,19 (d, 1H, ³J_{A-B}=3,6 Hz, H_{A}), 7,88 (m, 4H, H₄ et H_{4'}), 7,28 (m, 5H, H₆, H_{6'} et H_{E}), 7,20-7,00 (m, 7H, H_{B}, H_{C}, H₅, H₅, et H_{D}), 2,77 (t, 4H, CH₂ alk en α), 2,68 (t, 4H, CH₂ alk en β), 1,25-1,58 (m, 26H, CH₂ alk), 0,80 (m, 6H, CH₃ alk).
   **RMN ¹³C** : (300 MHz dans CDCl₃/MeOD (90/10), δppm)
   13,51 ; 22,24 ; 27,70 ; 28,80 ; 28,89 ; 29,21 ; 29,43 ; 29,45 ; 30,30 ; 30,35 ; 31,47 ; 118,52 ; 124,39 ; 124,43 ; 125,59 ; 125,92 ; 127,15 ; 127,48 ; 127,66 ; 128,22 ; 129,29 ; 131,10 ; 131,19 ; 135,13 ; 137,49 ; 138,07 ; 140,05 ; 190,72 ; 141,35 ; 191,90 ; 144,95 ; 151,27 ; 151,68 ; 154,69 ; 157,40 ; 157,78.

### Exemple 4 : Synthèse du complexe C4

Acide bis-tétrafluoroborate de ruthénium (II) [4'-(2-(3', 4'-dioctyl-2,2':5',2''-terthiophèn-5-yl)éthyl)-2,2':6',2''-terpyridine]-2,2':6',2''-terpyridine-4'-phosphonique (C4)

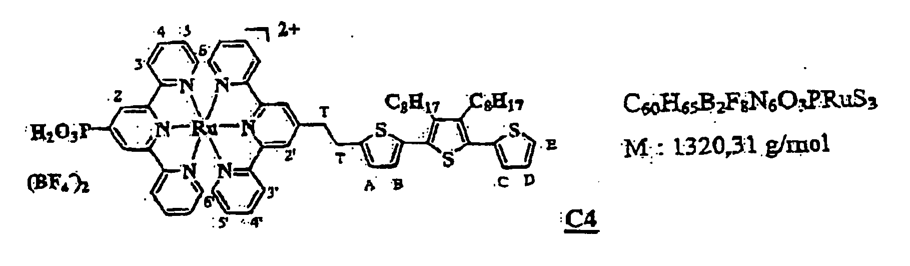

Synthèse du complexe intermédiaire C4a :
Bis-tétrafluoroborate de ruthénium (II) [4'-((3',4'-dioctyl-2,2' :5',2"-terthiophèn-5-yl)éthynyl)-2,2':6',2''-terpyridine]-2,2':6',2''-terpyridine-4'-phosphonate de diéthyle 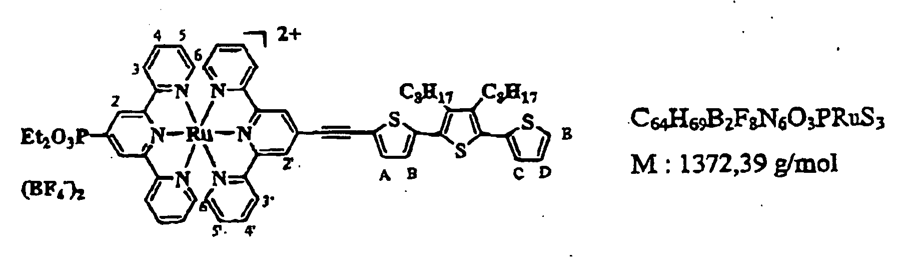
98 mg (0,2 mmol, 2 éq.) de 5-(éthynyl)-3',4'-dioctyl-2,2' :5',2"-terthiophène, 1 mL de triéthylamine distillée et 6,5 mL de DMF distillé sont introduits dans un tube scellé. Après 3 dégazages sous argon, 15 mg (0,02 mmol, 0,2 éq.) de [1,1'-bis(diphénylphosphino)ferrocène]dichloro-palladium et 4 mg (0,02 mmol, 0,2 éq) d'iodure de cuivre et 96 mg (0,1 mmol, 1 éq.) de bis tétrafluoroborate de ruthénium (II) [4'-brome-2,2' 6',2''-terpyridine]-2,2' :6',2''-terpyridine-4'-phosphonate de diéthyle sont ajoutés. Le mélange est chauffé à 90°C pendant 16 heures. Après refroidissement du milieu, l'ajout d'une solution saturée de tétrafluoroborate de sodium fait précipiter un solide rouge qui est filtré. Le produit obtenu est purifié par chromatographie sur silice (éludant : gradient d'eau et d'une solution de KNO₃ dans l'acétonitrile (de 100:0:0 à 70:20:1 acétone/H₂O/KNO₃) pour donner un solide rouge. Le complexe est dissous dans un minimum de méthanol et précipité par ajout d'une solution saturée de NaBF₄ pour donner un solide rouge (m : 50 mg, Rdt : 40%.
   **RMN ¹H** : (300 MHz dans CD₃CN, δppm)
   9,0 (d, ³J_{2-P}=13,5 Hz, 2H, H₂), 8, 93 (s, 2H, H_{2'}), 8,72 et 8,57 (2d, ³J₃₋₄ et ³J_{3'-4'}=7,8 Hz, 4H, H₃ et H_{3'}), 7,97 (m, 4H, H₄ et H_{4'}), 6,65 (d, ³J_{E-D}=3,9 Hz, 1H, H_{E}), 7,49 et 7,38 (m, 4H, H₆ et H_{6'}), 7,30-7,15 (m, 8H, H_{A}, H_{B}, H_{C}, H_{D}, H₅ et H_{5'}), 4,42 (m, 4H, OCH₂CH₃), 2,77 (t, 4H CH₂ alk en α), 2,68 (t, 4H, CH₂ alk en β), 1, 25-1,67 (m, 26H, OCH₂CH₃ et CH₂ alk), 0,88 (m, 6H, CH₃ alk).
   **RMN ¹³C** : (300 MHz dans CD₃CN, δppm)
   158,3 ; 156,6 156,5 ; 155,9 ; 153,8 ; 153,4 ; 142,7 ; 141,6 ; 141,4 ; 139,4 ; 139,3 ; 132,1 : 132,2 ; 129,4 ; 128,9 ; 128,7 ; 127,4 ; 126,1 ; 126,0 ; 125,7 ; 92,5 ; 91,4 ; 32,6 ; 31,3 ; 30,4 ; 29,9 ; 29 ;0 ; 23,4 ; 16,9 ; 16,8 ; 14,4.

Synthèse du complexe intermédiaire C4b :
Bis-tétrafluoroborate de ruthénium (II) [4'-(2-(3',4'-dioctyl-2,2' :5',2"-terthiophèn-5-yl)éthyl)-2,2':6',2''-terpyridine]-2,2' :6',2"-terpyridine-4'-phosphonate de diéthyle 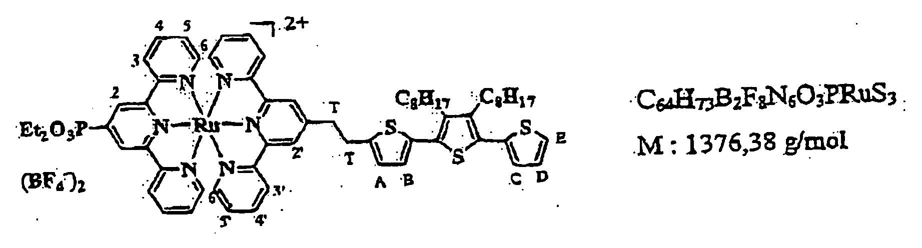
80 mg (0,06 mmol, 1 éq.) de complexe C4a dans 12 mL de méthanol distillé sont introduits dans un tricol. Après un dégazage sous argon, 40 mg (0,06 mmol, 3 éq.) de palladium sur charbon à 20% sont ajoutés avant l'introduction du dihydrogène. Le milieu réactionnel est agité vigoureusement pendant 14 heures à température ambiante. Le milieu est filtré sur célite puis le solvant est évaporé sous pression réduite. Le résidu est purifié sur Sephadex LH20 pour doner un solide rouge-orangé (m : 56 mg, Rdt : 70%).
   **RMN ¹H** : (300 MHz dans CD₃CN + 1 goutte de MeOD, δppm)
   8,93 (d, ³J_{2-P}=13,5 Hz, 2H, H₂), 8,62 (d, ³J₃₋₄ =7,8 Hz, 2H, H₃), 8,57 (s, 2H, H_{2'}), 8,40 (d, ³J_{3'-4'}=7,8 Hz, 4H, H_{3'}), 7,88 (m, 4H, H₄ et H_{4'}), 7,41 (d, ³J_{E-D}=3,9 Hz, 1H, H_{E}), 7,31 et 7,27 (m, 4H, H₆ et H_{6'}), 7,13-7,00 (m, 8H, H₄, H_{B}, H_{C}, H_{D}, H₅ et H₅'), 4,42 (m, 4H, OCH₂CH₃), 3,5 (m, 4H, 2CH_{2T}), 2,77 (t, 4H, CH₂ alk en α), 2,68 (t, 4H, CH₂ alk en β), 1,25-1,67 (m, 26H, OCH₂CH₃ et CH₂ alk), 0,88 (m, 6H, CH₃ alk).
   **RMN ¹³C :** (300 MHz dans CD₃CN + 1 goutte de MeOD, δppm)
   158,6 ; 158,3 ; 156,5 ; 155,0 ; 153,6 ; 152,8 ; 144,2 ; 141,0 ; 139,1 ; 138,8 ; 128,5 ; 128,1 ; 127,4 ; 127 ;0 ; 126,7 ; 125,8 ; 125,1 ; 64,4 ; 38,2 ; 32,3 ; 31,1 ; 30,1 ; 29,7 ; 28,4 ; 23,1 ; 16,9 ; 16,8 ;: 14,2.

### Synthèse finale du complexe C4

Dans un tube scellé, 50 mg (0,36 mmol, 1 éq.) de complexe C4b sont introduits dans 3 mL de DMF fraîchement distillé et 290 mg (2 mmol, 50 éq.) de bromotriméthylsilane anhydre sont alors précautionneusement ajoutés sous argon. Le milieu réactionnel est chauffé à 50°C pendant 36 heures sous argon. Après retour à température ambiante, l'ajout de dichlorométhane entraîne la précipitation d'un solide qui est filtré, lavé au dichlorométhane et séché sous vide. Le complexe est dissous dans un minimum de méthanol et précipité par ajout d'une solution saturée de NaBF₄ pour donner un solide rouge purifié sur colonne Sephadex LH20 (m : 40 mg, Rdt : 90%).
**RMN ¹H** : (300 MHz dans MeOD + 1 goutte de CD₃CN, δ ppm)
9,16 (d, 2H, ³J_{2-P}=13,5 Hz, H₂), 8,67 (d, 2H, ³J₃₋₄=7,8 Hz, H₃), 8,57 (s, 2H, H_{2'}), 8,40 (d, 4H, ³J_{3'-4'} =7,8 Hz, H_{3'}) ; 7,83 (m, 4H, H₄ et H_{4'}), 7,39 (d, ³J_{E-D} =3,9 Hz, 1H, H_{E}), 7,33 et 7,27 (m, 4H, H₆ et H_{6'}), 7,13-7,00 (m, 8H, H_{A}, H_{B}, H_{C}, H_{D}, H₅ et H_{5'}), 3,5 (m, 4H, 2CH_{2T}), 2,77 (t, 4H CH₂ alk en α), 2,68 (t, 4H, CH₂ alk en β), 1,25-1,67 (m, 20H, CH₂ alk), 0,88 (m, 6H, CH₃ alk).

### Exemple 5 : Fabrication de cellules photovoltaïques

Une plaque de verre conductrice (SnO₂ dopé au fluor), sur laquelle a été déposée une couche de TiO₂ dense, est lavée à l'eau, rincée à l'acétone et à l'éthanol puis séchée sous un flux d'azote. Ensuite, un film transparent de nanoparticules de TiO₂ est déposé par enduction centrifuge à partir d'une solution de dioxyde de titane fournie par la société Solaronix. Après 5 minutes de séchage à l'air ambiant, le substrat est chauffé à 450°C pendant 30 minutes. L'épaisseur du film de TiO₂ obtenu est d'environ 1,5 µm.

La plaque est immergée dans une solution de complexe colorant (conditions dans le tableau suivant) pendant 16 heures avant d'être rincée avec le même solvant, et séchée.

| colorant | solvant | concentration | température |
|---|---|---|---|
| **5** | THF | 2.10⁻³M | 60°C |
| **12'** | EtOH/MeOH (90/10) | 3.10⁻⁴M | 25°C |

Ensuite, une solution à 35 g.L⁻¹ de poly(3-octylthiophène) dans le toluène est déposée par enduction centrifuge. Pour terminer, la cellule est recouverte d'une couche d'or de 200 nm déposée par PVD.

On prépare des cellules photovoltaïques de manière analogue, avec les autres complexes préparés ci-dessus et les autres complexes, colorants, testés cités.

### Exemple 6 : Performances des cellules photovoltaïques comprenant les complexes 5 et 12'

Les résultats des performances photovoltaïques des cellules sèches basées sur les colorants 12' et sur les complexes 5 sont consignés dans le tableau 1 ci-dessous.

**Tableau 1 : *Moyenne de 5 mesures indépendantes, Voc = tension de circuit ouvert, Isc = courant de court circuit et ff = facteur de forme.**

| Complexe | *Voc (V) | *Isc (MA.cm²) | *ff (%) | *η (%) |
|---|---|---|---|---|
| **5** | 0,71 | 0,53 | 60 | 0,22 |
| **12'** | 0,68 | 0,30 | 26 | 0,05 |

Ces mesures montrent clairement que la mise en oeuvre dans des cellules photovoltaïques d'un complexe colorant qui comprend un lien chimique entre le groupe sensibilisateur et la chaîne de polymère conducteur comme c'est le cas dans le complexe 5 conforme à l'invention, permet de quadrupler le rendement de la cellule photovoltaïque par rapport à une cellule photovoltaïque où l'on met en oeuvre un complexe-colorant non-conforme à l'invention et ne comprenant pas un tel lien chimique, un tel espaceur, entre le groupe sensibilisateur et le polymère conducteur.

Le courant débité, le photo-potentiel et le facteur de forme sont tous les trois améliorés par la mise en oeuvre du complexe et du nouveau matériau hybride selon l'invention.

Dans les exemples 7 et 8 qui suivent, on décrit les performances de cellules photovoltaïques comprenant les composés C1, C3 et C4 qui sont des sensibilisateurs de type bis terpyridinique et les composés C9, C12 et C13 (composé 5 de l'exemple 1) qui sont des sensibilisateurs de type bipyridinique terpyridinique.

Les composés C1 et C9 sont utilisés, pour comparaison, comme analogues non greffés des composés respectivement « C3 et C4 » et « C12 et C13 ». Les composés C3 et C12 ne comportent pas d'espaceur tandis que les composés C4 et 13 comportent un espaceur et sont donc conformes à l'invention.

### Exemple 7 : Performance des cellules photovoltaïques comprenant des complexes sensibilisateurs de type bis terpyridinique : à savoir les composés C1, C3 et C4 (27).

| Composés | Référence | Voc | Isc | ff | η |
|---|---|---|---|---|---|
| 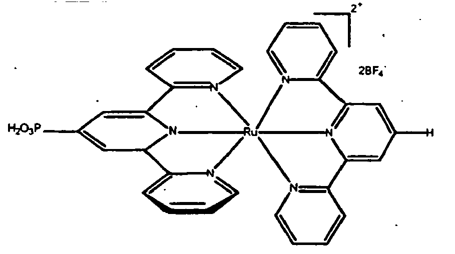 | C1 | 0,7 | 0,15 | 35 | 0,04 |
| 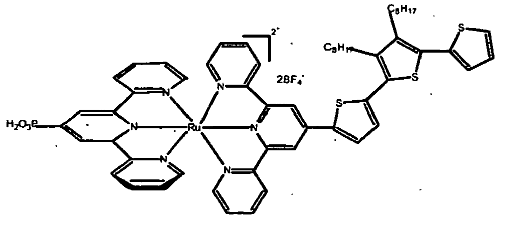 | C3 | 0,78 | 0,13 | 33 | 0,03 5 |
| 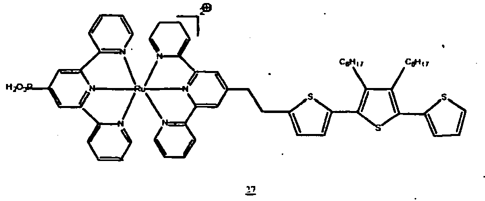 | C4 | 0,95 | 0,14 | 35 | 0,05 |

C1 est un colorant de référence utilisé pour réaliser des cellules selon la technique classique, c'est-à-dire un dépôt de polymère par « spin coating ».

Le complexe C3, sur lequel est greffé un oligomère de polythiophène sans espaceur tel que décrit dans le document [31] conduit à une chute de rendement de 12,5% par rapport à C1.

Le complexe C4 conforme à l'invention, sur lequel est greffé l'oligomère de polythiophène à l'aide d'un espaceur conduit à une augmentation de rendement de 25% par rapport à C1.

### Exemple 8 : Performance de cellules photovoltaïques comprenant des complexes sensibilisateurs de type bipyridinique terpyridinique

| Composés | Référence | Voc | Isc | ff | η |
|---|---|---|---|---|---|
| 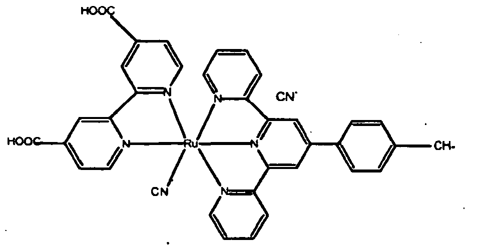 | C9 | 0,58 | 0,34 | 64 | 0,13 |
| 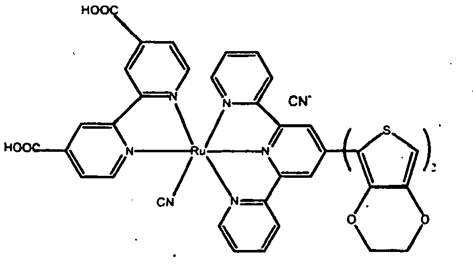 | C12 | 0,77 | 0,27 | 33 | 0,068 |
| 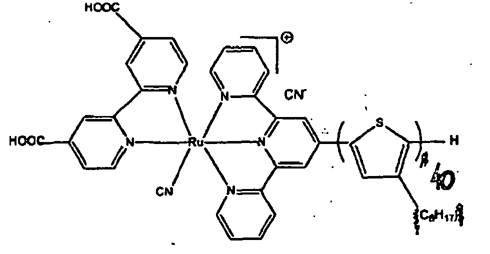 | C13 (5) (n=40 et R=n-octyle) | 0,79 | 0,51 | 66 | 0,27 |

Le sensibilisateur C9 est un colorant de reference utilise pour réaliser des cellules selon la technique classique, c'est-à-dire un dépôt de polymère par « spin coating ».

Le complexe C12, sur lequel est greffé un oligomère de polythiophène sans espaceur tel que décrit dans le document [31] conduit à une chute de rendement de 47,7% par rapport à C9.

Le complexe C13 (5) conforme à l'invention, sur lequel est greffé l'oligomère de polythiophène à l'aide d'un espaceur conduit à une augmentation de rendement de 107,6% par rapport à C9.

Au regard des exemples 7 et 8, on peut effectuer les remarques suivantes :
C3 et C12 ne disposent pas d'espaceur. Les résultats photovoltaïques de ces matériaux sont plus faibles que ceux de leurs homologues non greffés. Ceci mohntre que le greffage direct est néfaste contrairement à ce qui est exprimé dans le document [31].
C4 et C13 (5) disposent d'un espaceur. Les résultats photovoltaïques de ces matériaux sont supérieurs à ceux de leurs homologues non greffés, ce qui montre l'intérêt du greffage avec espaceur.

### RÉFÉRENCES

[1] Hagfeld, A. : Grätzel, M. Chem. Rev. 1995, 95, 49.
[2] Kalyanasundaram, K. ; Grätzel, M. Coord. Chem. Rev. 1998, 177, 347-414.
[3] O'Regan, B. ; Grätzel, M. Nature 1991, 353, 737-740.
[4] Duffy, N.W. ; Peter, L.M. ; Rajapaske, R.M.G. ; Wijayantha, K.G.U., J. Phys. Chem. B. 2000, 104, 8916-8919.
[5] Perera, V.P.S. ; Tennakone, K. Sol. Energy Mater. Sol. Cells 2003, 79, 249-255.
[6] O'Regan, B. ; Schwartz, D.T. Chem. Mater. 1998, 10, 1501-1509.
[7] Kumara, G.R.R.A. ; Konno, A. ; Senadeera, G.K.R. ; Jayaweera, P.V.V. ; de Silva, D.B.R.A. ; Tennakone, K. Sol. Energy Mater. Sol. Cells 2001, 69, 195-199.
[8] Hao, Y. ; Yang, M. ; Li, W. ; Qiao, X. ; Zhang, L. ; Cai, S. Sol. Energy Mater. Sol., Cells 2000, 60, 349-359.
[9] Murakoshi, K. ; Kogure, R. ; Wada, Y. ; Yanagida, S. Chem. Lett. 1997, 471-472.
[10] Murakoshi, K. ; Kogure, R. ; Wada, Y. ; Yanagida, S. Sol. Energy Mater. Sol. Cells 1998, 55, 113-115.
[11] Nogueira, A.F. ; Longo, C. ; De Paoli, M.A. Coord. Chem. Rev. 2004, 248, 1455-1468.
[12] Gebeyehu, D. ; Brabec, C.J. ; Padinger, F. ; Fromherz, T. ; Spiekermann, S. ; Vlachopoulos, N. ; Kienberger, F. ; Schindler, H. ; Sariciftci, N.S. Synth. Metals 2001, 121, 1549-1550.
[13] Gebeyehu, D. ; Brabec, C.J. ; Sariciftci, N.S., Thin Solid Films 2002, 403-404, 271-274.
[14] Gebeyehu, D. ; Brabec, C.J. ; Sariciftci, N.S. ; Vengeneugden, D. ; Kiebooms, R. ; Vanderzande, D. ; Kienberger, F. ; Schindler, H. Synth. Metals 2002, 125, 279-287.
[15] Kajihara, K. ; Tanaka, K. ; Hirao, K. ; Soga, N. Jpn J. Appl. Phys. 1997, 36, 5537-5542.
[16] Kaneko, M. ; Takayama, K. ; Pandey, S.S. ; Takashima, W. ; Endo, T. ; Rikukawa, M. ; Kaneto, K. Synth. Metals 2001, 121, 1537-1538.
[17] Nagamatsu, S. ; Pandey, S.S. ; Takashima, W. ; Endo, T. ; Rikukawa, M. ; Kaneto, K. Synth. Metals 2001, 121, 1563-1564.
[18] Saito, Y. ; Azechi, T. ; Kitamura, T. ; Hasegawa, Y. ; Wada, Y. ; Yanagida, S., Coord. Chem. Rev. 2004, 248, 1469-1478.
[19] Senadeera, G.K.R. ; Nakamura, K. ; Kitamura, T. ; Wada, Y. ; Yanagida, S. Appl. Phys. Lett. 2003, 83, 5470-5472.
[20] Spiekermann, S. ; Smestad, G. ; Kowalik, J. ; Tolbert, L.M. ; Grätzel, M. Synth. Metals, 2001, 121, 1603-1604.
[21] Takayama, K. ; Kaneko, M. ; Pandey, S.S. ; Takashima, W. ; Kaneto, K. Synth. Metals 2001, 121, 1565-1566.
[22] Bach, U. ; Lupo, D. ; Comte, P. ; Moser, J.E. ; Weissortel, F. ; Salbeck, J. ; Spreitzer, H., Grâtzel, M Nature 1998, 395, 583-585.
[23] Kinoshita, M ; Fujii, N. ; Tsuzuki, T. Shirota, Y. Synth. Metals 2001, 121, 1571-1572.
[24] Haridas, K.R. ; Ostrauskaite, J. ; Thelakkat, M. ; Heim, M. ; Bilke, R. ; Haarer, D. Synth. Metals 2001, 121, 1573-1574.
[25] Jager, C. ; Bilke, R. ; Heim, M. ; Haarer, D. ; Karickal, H. ; Thelakkat, M. Synth. Metals 2001, 121, 1543-1544.
[26] Bach, U. ; Tachibana, Y. ; Moser, J.E. ; Haque, S.A. ; Durrant, J.R. ; Graetzel, M. ; Klug, D.R. J. Am. Chem. Soc. 1999, 121, 7445-7446.
[27] Jortner, J. ; Ratner, M. Molecular Electronics ; Blackwell Science : London, 1997.
[28] Bixon, M. ; Jortner, J. Electron Transfer - From isolated molecules to biomolecules ; John Wiley & Sons : New York 1999.
[29] Odobel, F. ; Zabri, H. Inorg. Chem. 2005, 44, 5600-5611.
[30] Jeffries-El, M. ; Sauve, G. ; McCullough, R.D. Adv. Mater. 2004, 16, 1017-1019.
[31] Krebs, F.C ; Biancarlo M. ; Solar Energy Materials and Solar Cells 90 (006), 142-165, "available on line 18 April 2005".
[32] FR-A-2 862 429.

## Revendications

1. Complexe de formule (I) 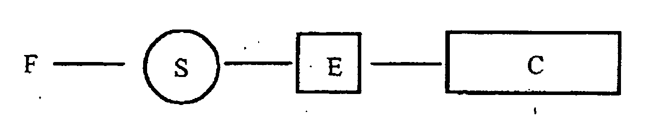 dans laquelle :
- F représente un ou plusieurs groupes aptes à se greffer chimiquement à un substrat en céramique oxyde poreuse semiconductrice ;
- S représente un groupe sensibilisateur d'une céramique oxyde poreuse semiconductrice ;
- C est un polymère conducteur de l'électricité ;
- E est un groupe espaceur déconjuguant permettant d'isoler électriquement le sensibilisateur (S) du polymère conducteur de l'électricité (C).

2. Complexe selon la revendication 1, dans lequel ladite céramique oxyde poreuse semiconductrice est une céramique semiconductrice de type n ou p, à large bande interdite.

3. Complexe selon l'une quelconque des revendications précédentes dans lequel le ou les groupes F aptes à se greffer chimiquement à la céramique oxyde poreuse semiconductrice sont :
- COOR¹ avec R¹ représentant un atome d'hydrogène, un groupe alkyle comprenant de 1 à 30 atomes de carbone ou un groupe phényle ;
- COCl ;
- COCH₂CO-R¹ avec R¹ représentant un atome d'hydrogène, un groupe alkyle comprenant de 1 à 30 atomes de carbone ou un groupe phényle ;
- PO(OH)₂, -PO(OR²)(OH) ou -PO(OR²)(OR³) avec R² et R³, identiques ou différents, représentant un groupe alkyle comprenant de 1 à 30 atomes de carbone ou un groupe phényle ;
- CO(NHOH) ;
- M(OR⁴)ₘ₋ₓZₓ avec x étant un entier allant de 1 à (m-1), M étant un métal ou un métalloïde, m étant un degré d'oxydation de M, R⁴ représentant un atome d' hydrogène, un groupe alkyle comprenant de 1 à 30 atomes de carbone, un groupe phényle, un cation métallique monovalent, ou un groupe de formule N⁺R¹₄, avec R¹ représentant un atome d'hydrogène, un groupe alkyle comprenant de 1 à 30 atomes de carbone, ou un groupe phényle, et Z représente un atome d'hydrogène, un groupe alkyle comprenant de 1 à 30 atomes de carbone, un groupe phényle ou un atome d'halogène;
- SO₃M' avec M' représentant un atome d'hydrogène, un cation métallique monovalent ou un groupe de formule N⁺R¹₄ avec R¹ représentant un atome d' hydrogène, un groupe alkyle comprenant de 1 à 30 atomes de carbone ou un groupe phényle ;
- B(OM')₂ avec M' représentant un atome d'hydrogène, un cation métallique monovalent ou un groupe de formule N⁺R¹₄ avec R¹ représentant un atome d'hydrogène, un groupe alkyle comprenant de 1 à 30 atomes de carbone ou un groupe phényle ;
- OH ;
et les combinaisons de ceux-ci.

4. Complexe selon l'une quelconque des revendications précédentes, dans lequel ledit groupe sensibilisateur S est choisis parmi les complexes polypyridiniques avec un métal de transition et les cations organiques tels que les phtalocyanines, les coumarines, et les cyanines.

5. Complexe selon la revendication 4, dans lequel ledit groupe sensibilisateur est un groupe de formule : 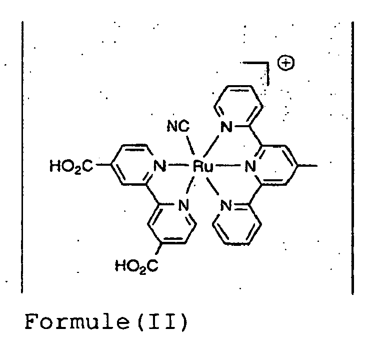 ou de formule

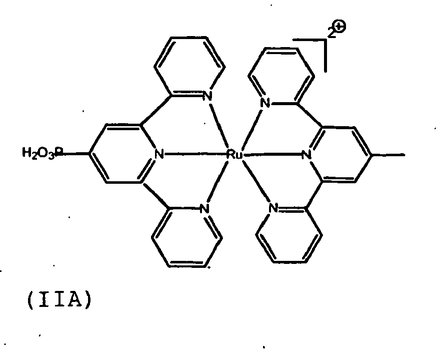

6. Complexe selon l'une quelconque des revendications précédentes, dans lequel ledit polymère conducteur de l'électricité C est choisi parmi les poly(acétylène)s, les poly(p-phénylène)s, les poly(p-phénylène vinylène)s), les poly(p-phénylène sulfure)s, les poly(pyrrole)s, les polythiophène)s, les poly(alkyl thiophène)s, les poly(dialkylthiophène)s, les poly(furane)s, les poly(alcoxy thiophène)s, les poly(azulène)s, les poly(azine)s, les poly(aniline)s, les poly(cyanophénylène vinylène)s, les poly(parapyridyl vinylène)s, les poly(dioxythiophène)s ("PEDOT"),
et les mélanges et/ou combinaisons et/ou copolymères de ceux-ci.

7. Complexe selon la revendication 6, dans lequel ledit polymère conducteur de l'électricité C est un polymère régiorégulier.

8. Complexe selon la revendication 7, dans lequel ledit polymère conducteur de l'électricité est choisi parmi les polymères suivants : 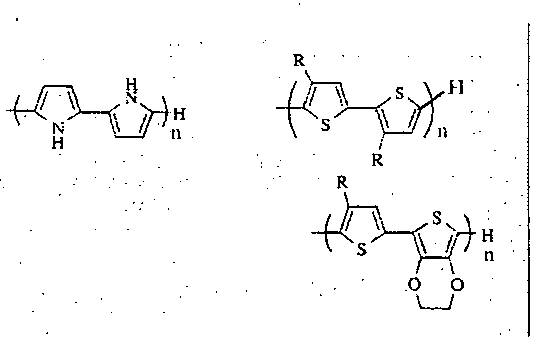 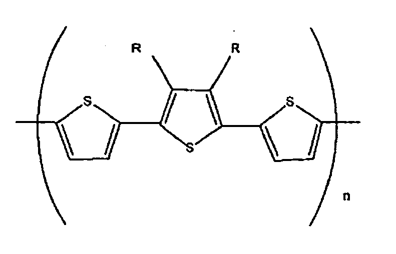 où n représente un nombre entier de 1 à 1 000, de préférence de 5 à 100, et R représente un groupe choisi parmi les groupes alkyle de 1 à 24C, de préférence de 4 à 12C, par exemple R est un groupe n-octyle, et les groupes alcoxy de 1 à 24C, de préférence de 4 à 12C.

9. Complexe selon l'une quelconque des revendications précédentes, dans lequel le groupe espaceur déconjuguant E est choisi parmi les groupes : 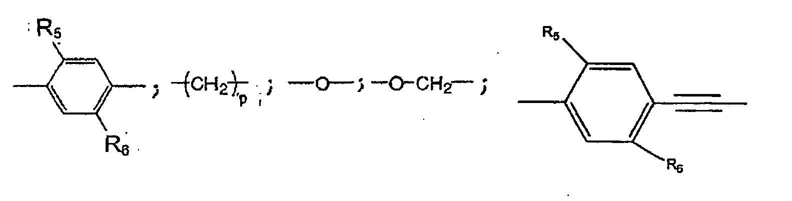 où R₅ et R₆ identiques ou différents sont choisis parmi les groupes alkyles de 1 à 24C, de préférence de 1 à 12C, et les groupes alcoxy de 1 à 24C, de préférence de 1 à 12C ; p est un nombre entier de 1 à 20, de préférence de 1 à 4.

10. Complexe selon la revendication 1 qui répond à la formule (III) ou (IIIA) suivante : 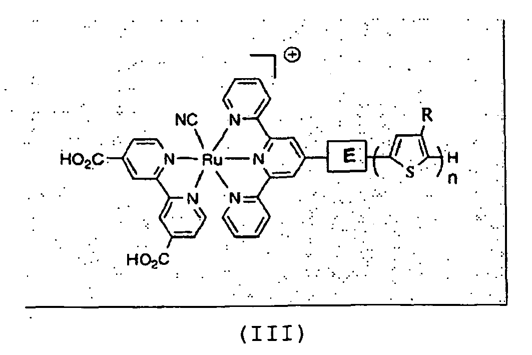 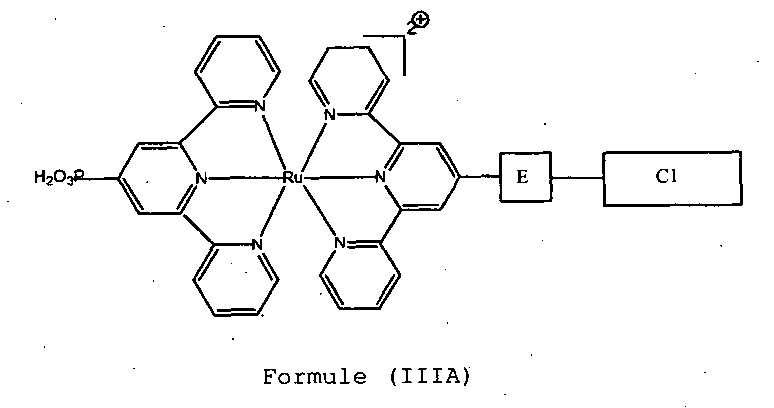 dans lesquelles n représente un nombre entier de 1 à 1 000, de préférence de 5 à 100 ; R représente un groupe alkyle de 1 à 24C, de préférence de 4 à 12C, ou un groupe alcoxy de 1 à 24C, de préférence de 4 à 12C ; E est choisi parmi les groupes 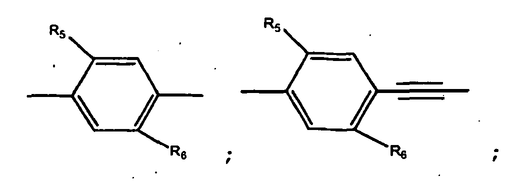 et -(CH₂)₂-,
R₅ et R₆ identiques ou différents sont choisis parmi les groupes alkyle de 1 à 24C, de préférence de 1 à 12C, et les groupes alcoxy de 1 à 24C, de préférence de 1 à 12C ; et C1 représente 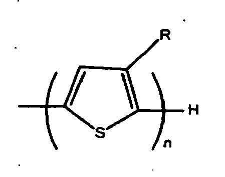 ou

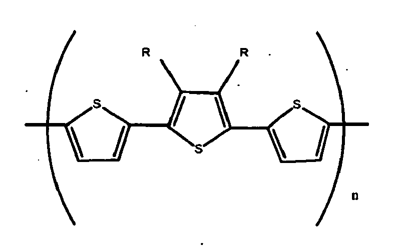

11. Procédé de préparation d'un complexe de formule (III) selon la revendication 10, où E représente -(CH₂)₂-, ou 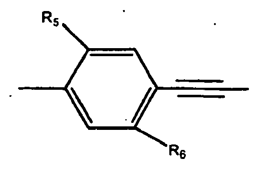 dans lequel on réalise les étapes successives suivantes :
a) - on fait réagir un composé de formule 1 suivante, ou un composé de formule 12 suivante : 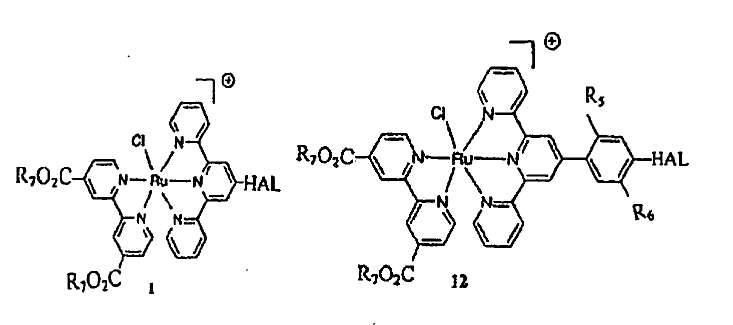 avec un composé de formule 2 suivante : 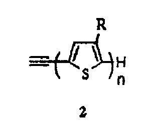 dans lesquelles n représente un nombre entier de 1 à 1 000, de préférence de 5 à 100, et R, R₅, R₆ et R₇, identiques ou différents, représentent un groupe alkyle de 1 à 24C, de préférence de 4 à 12C pour R, et de préférence de 1 à 12C pour R₅, R₆ et R₇, ou un groupe alcoxy de 1 à 24C, de préférence de 4 à 12C pour R, et de préférence de 1 à 12C pour R₅, R₆ et R₇, de préférence encore R₇ est un groupe éthyle, et HAL représente un atome d'halogène, de préférence un atome de Br ; selon une réaction de SONOGASHIRA, dans un mélange de DMF/THF, en présence d'un système catalytique comprenant de l'iodure de cuivre, du [1,1'-bis(diphénylphosphino) ferrocène] dichloro-palladium (II) et de la triéthylamine, pour obtenir respectivement un composé de formule 3 suivante, ou un composé de formule 13 suivante : 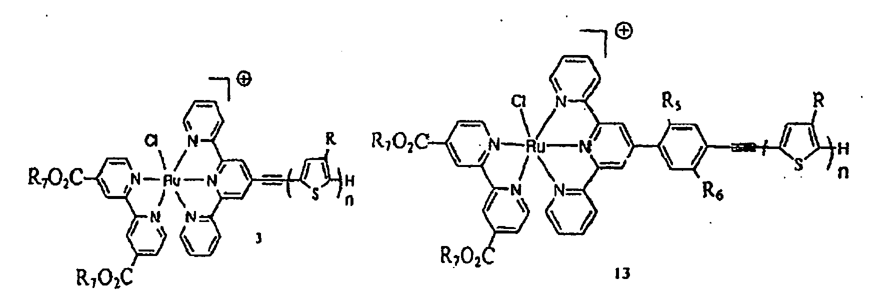
b) - on fait réagir le composé de formule 3 avec de l'hydrogène dans le THF en présence de palladium sur charbon pour obtenir un composé de formule 4 suivante : 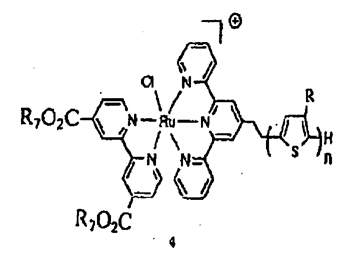
c) - on fait réagir le composé de formule 4 ou le composé de formule 13 dans un mélange de THF/H₂O avec KCN/LiOH pour obtenir respectivement un composé de formule 5 suivante ou de formule 14 suivante :

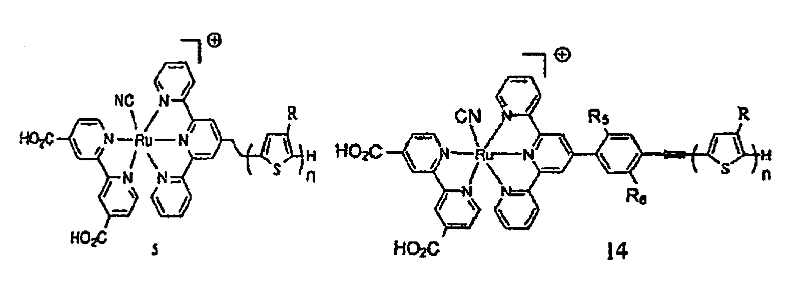

12. Procédé selon la revendication 11, dans lequel le composé de formule 12 est préparé par réaction d'un composé de formule 10 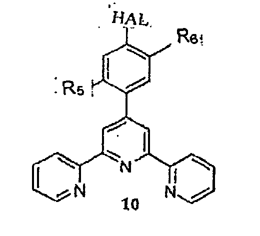 où HAL représente un atome d'halogène, de préférence un atome de Br
avec un composé de formule 11

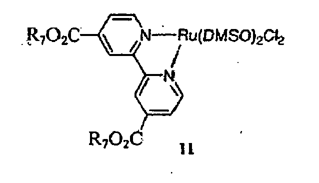

13. Composé de formule 10 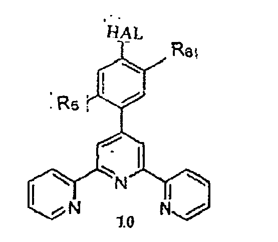 où HAL représente un atome d'halogène, de préférence un atome de Br et où
R₅ et R₆ identiques ou différents sont choisis parmi les groupes alkyle de 1 à 24C, de préférence de 1 à 12C, et les groupes alcoxy de 1 à 24C, de préférence, de 1 à 12C.

14. Matériau hybride inorganique-organique semiconducteur P-N comprenant un substrat en céramique oxyde poreuse auquel est greffé chimiquement un complexe de formule (I), selon l'une quelconque des revendications 1 à 10.

15. Matériau selon la revendication 14, dans lequel la céramique oxyde poreuse est choisie parmi des céramiques à base de métaux de transition choisis parmi Ti, V, Cr, Mn, Fe, Co, Ni, Cu, Zn, Y, Zr, Nb, Mo, Ru, Rh, Pd, Ag, Cd, Hf, Ta, W, Re, Os, Ir, Pt ou à base de lanthanides, tels que La, Ce, Pr, Nd, Sm, Eu, Gd, Tb, Dy, Er, et Yb ou à base d'éléments du groupe IIIA de la classification périodique choisis parmi Al, Ga, In et Tl ou à base d'éléments du groupe IVA de la classification périodique choisis parmi Si, Ge, Sn et Pb ou à base d'éléments du groupé VIA de la classification périodique choisis parmi Se et Te.

16. Matériau semi-conducteur selon l'une quelconque des revendications 14 et 15, dans lequel la céramique oxyde poreuse est une céramique mésoporeuse.

17. Matériau semi-conducteur selon la revendication 16, dans lequel la céramique mésoporeuse est mésostructurée.

18. Matériau semi-conducteur selon l'une quelconque des revendications 14 à 17, dans lequel la céramique est du dioxyde de titane TiO₂.

19. Procédé de préparation du matériau hybride inorganique-organique semiconducteur selon l'une quelconque des revendications 14 à 18, dans lequel on imprègne une céramique oxyde poreuse semiconductrice par une solution organique ayant un ou plusieurs complexes de formule (I).

20. Cellule photovoltaïque comprenant :
- une première électrode collectrice de courant (dite « électrode de travail ») ;
- une seconde électrode (dite « contre-électrode de-travail ») ;
- une zone semi-conductrice constituée d'un matériau tel que défini selon l'une quelconque des revendications 14 à 18, ladite zone étant disposée entre ladite première électrode et ladite seconde électrode.

## Claims

1. Complex having the formula (I) 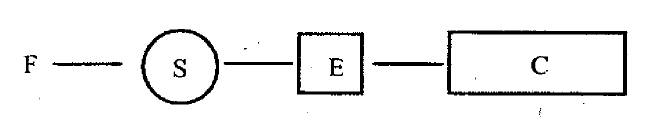 wherein:
- F represents one or more groups capable of being chemically grafted on a semiconductive porous oxide ceramic substrate;
- S represents a semiconductive porous oxide ceramic sensitising group;
- C is an electricity conducting polymer;
- E is a deconjugating spacer group enabling the electrical isolation of the sensitiser (S) from the electricity conducting polymer (C).

2. Complex according to claim 1, wherein said semiconductor porous oxide ceramic is a type n or p semiconductor ceramic, with a wide forbidden band.

3. Complex according to any one of the preceding claims, wherein the F group(s) capable of being chemically grafted on the semiconductive porous oxide ceramic are:
- COOR¹ where R¹ represents a hydrogen atom, an alkyl group comprising 1 to 30 carbon atoms or a phenyl group;
- COCl;
- COCH₂CO-R¹ where R¹ represents a hydrogen atom, an alkyl group comprising 1 to 30 carbon atoms or a phenyl group;
- PO(OH)₂, -PO(OR²)(OH) or -PO(OR²)(OR³), where R² and R³, identical or different, represent an alkyl group comprising 1 to 30 carbon atoms or a phenyl group;
- CO(NHOH);
- M(OR⁴)ₘ₋ₓZₓ where x is an integer ranging from 1 to (m-1), M is a metal or a metalloid, m is a degree of oxidation of M, R⁴ represents a hydrogen atom, an alkyl group comprising 1 to 30 carbon atoms, a phenyl group, a monovalent metallic cation, or a group having the formula N⁺R¹₄, where R¹ represents a hydrogen atom, an alkyl group comprising 1 to 30 carbon atoms, or a phenyl group, and Z represents a hydrogen atom, an alkyl group comprising 1 to 30 carbon atoms, a phenyl group or a halogen atom;
- SO₃M' where M' represents a hydrogen atom, a monovalent metallic cation, or a group having the formula N⁺R¹₄, where R¹ represents a hydrogen atom, an alkyl group comprising 1 to 30 carbon atoms, or a phenyl group;
- B(OM')₂ where M' represents a hydrogen atom, a monovalent metallic cation, or a group having the formula N⁺R¹₄, where R¹ represents a hydrogen atom, an alkyl group comprising 1 to 30 carbon atoms, or a phenyl group;
- OH;
and combinations thereof.

4. Complex according to any one of the preceding claims, wherein said sensitising group S is selected from polypyridine complexes with a transition metal and organic cations such as phthalocyanins, coumarins, and cyanins.

5. Complex according to claim 4, wherein said sensitising group is a group according to the formula: 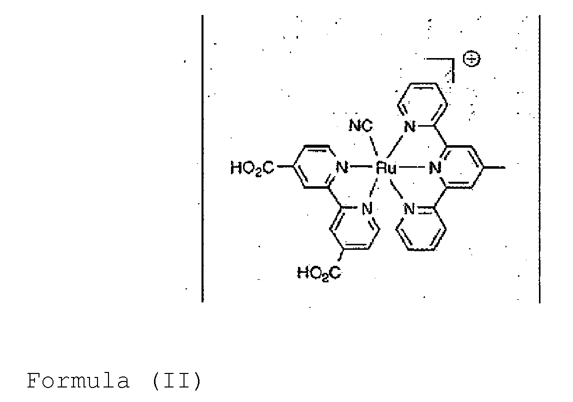 or according to the formula

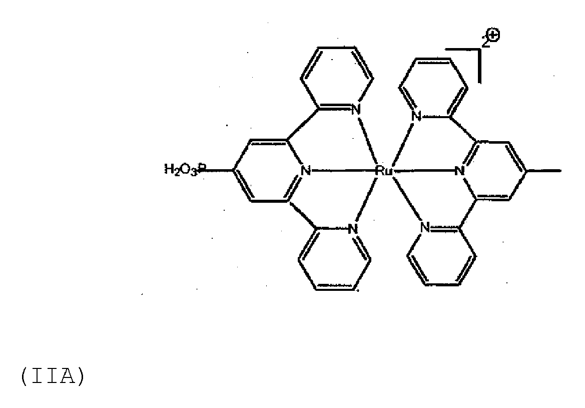

6. Complex according to any of the above claims, wherein said electricity conducting polymer C is selected from poly(acetylene)s, poly(p-phenylene)s, poly(p-phenylene vinylene)s, poly(p-phenylene sulphide)s, poly(pyrrole)s, poly(thiophene)s, poly(alkyl thiophene)s, poly(dialkylthiophene)s, poly (furan) s, poly (alcoxy thiophene) s, poly (azulene) s, poly(azine)s, poly(aniline)s, poly(cyanophenylene vinylene)s, poly(parapyridyl vinylene)s, poly(dioxythiophene)s ("PEDOT"),
and mixtures and/or combinations and/or copolymers thereof.

7. Complex according to claim 6, wherein said electricity conducting polymer C is a regioregular polymer.

8. Complex according to claim 7, wherein said electricity conducting polymer is selected from the following polymers: 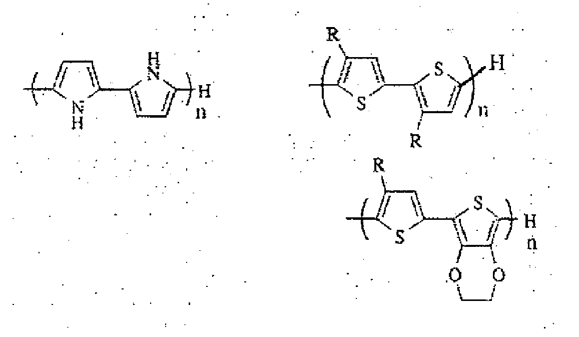 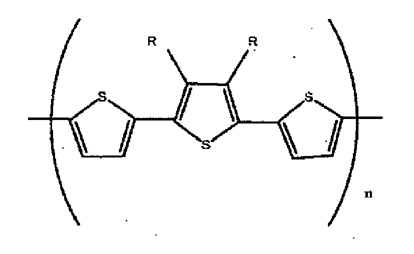 where n represents an integer from 1 to 1000, preferentially from 5 to 100, and R represents a group selected from the alkyl groups having 1 to 24C, preferentially 4 to 12C, for example R is an n-octyl group, and the alcoxy groups having 1 to 24C, preferentially 4 to 12C.

9. Complex according to any one of the preceding claims, wherein the deconjugating spacer group E is selected from the following groups: 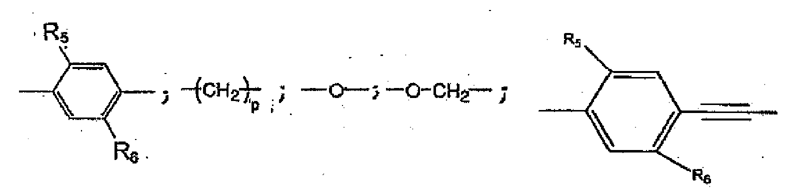 where R₅ and R₆, identical or different, are selected from the alkyl groups having 1 to 24C, preferentially 1 to 12C, and the alcoxy groups having 1 to 24C, preferentially 1 to 12C; p is an integer from 1 to 20, preferentially from 1 to 4.

10. Complex according to claim 1 complying with the following formula (III) or (IIIA): 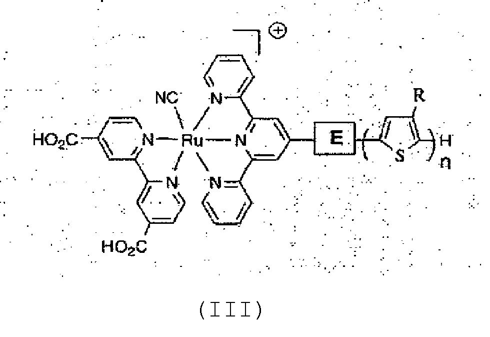 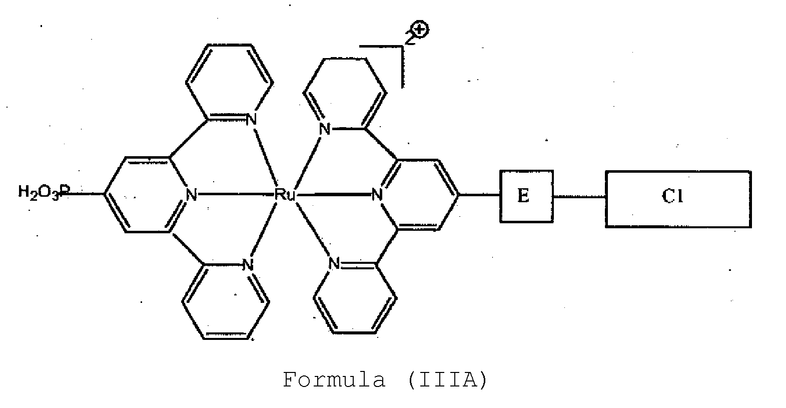 wherein n represents an integer from 1 to 1000, preferentially from 5 to 100; R represents an alkyl group having 1 to 24C, preferentially 4 to 12C, or an alcoxy group having 1 to 24C, preferentially from 4 to 12C; E is selected from the groups 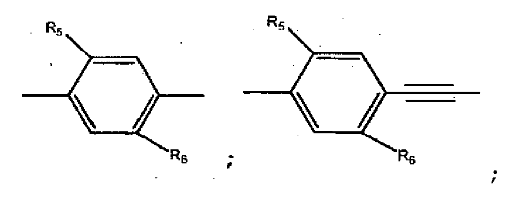 and -(CH₂)₂-,
R₅ and R₆, identical or different, are selected from the alkyl groups having 1 to 24C, preferentially 4 to 12C, and the alcoxy groups having 1 to 24C, preferentially 4 to 12C; and C1 represents 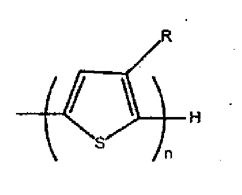 or

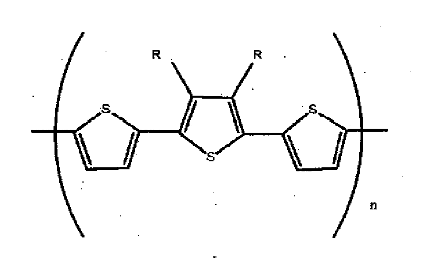

11. Method for preparing a complex according to formula (III) according to claim 10, where E represents -(CH₂)₂-, or 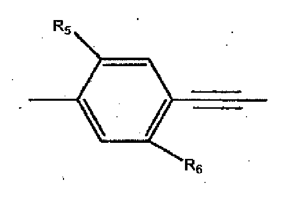 wherein the following successive steps are performed:
a) - a compound according to formula 1 hereinafter, or a compound according to formula 12 hereinafter is reacted: 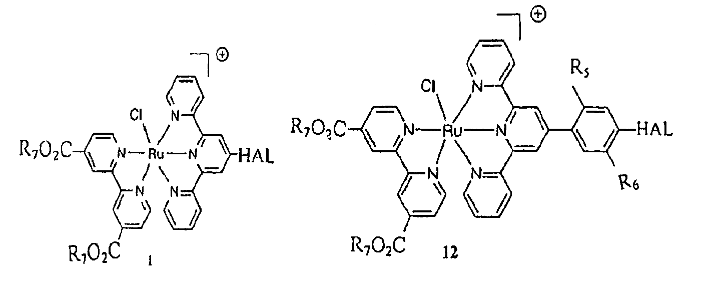 with a compound according to formula 2 hereinafter: 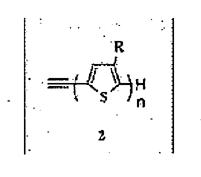 wherein n represents an integer from 1 to 1000, preferentially from 5 to 100, and R, R₅, R₆ and R₇, identical or different, represent an alkyl group having 1 to 24C, preferentially 4 to 12C for R, and preferentially 1 to 12C for R₅, R₆ and R₇, or an alcoxy group having 1 to 24C, preferentially 4 to 12C for R, and preferentially 1 to 12C for R₅, R₆ and R₇, more preferentially R₇ is an ethyl group, and HAL represents a halogen atom, preferentially a Br atom; according to a SONOGASHIRA reaction, in a DMF/TMF mixture, in the presence of a catalytic system comprising copper iodide, [1,1'-bis(diphenylphosphino) ferrocène] dichloro-palladium (II) and triethylamine, to obtain a compound according to formula 3 hereinafter, or a compound according to formula 13 hereinafter: 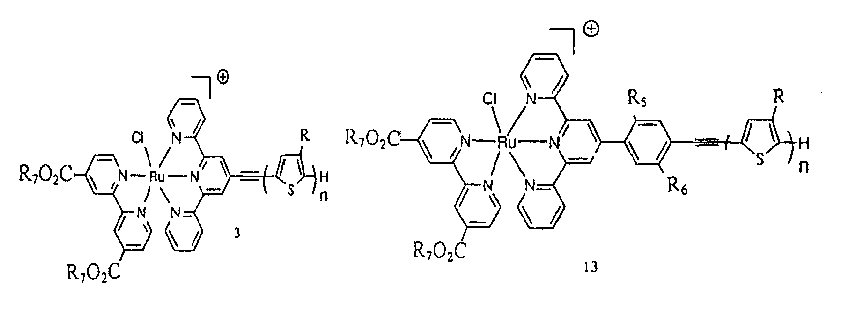
b) - the compound according to formula 3 is reacted with hydrogen in THF in the presence of palladium on carbon to obtain a compound according to formula 4 hereinafter: 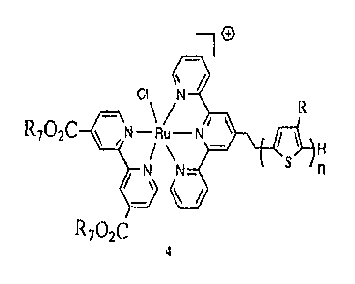
c) - the compound according to formula 4 or the compound according to formula 13 is reacted in a THF/H₂O mixture with KCN/LiOH to obtain a compound according to formula 5 hereinafter or formula 14 hereinafter:

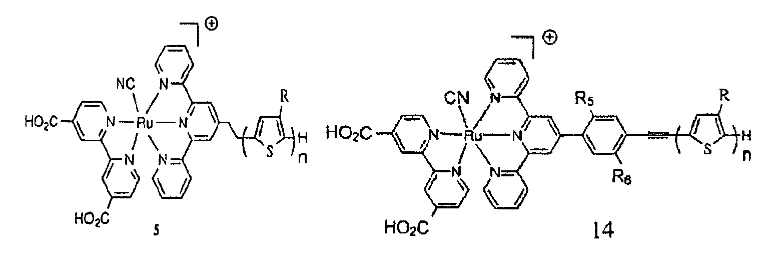

12. Method according to claim 11, wherein the compound according to formula 12 is prepared by reacting a compound according to formula 10 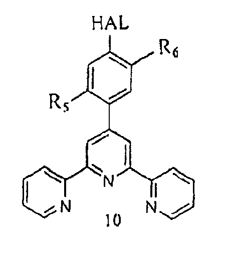 where HAL represents a halogen atom, preferentially a Br atom
with a compound according to formula 11

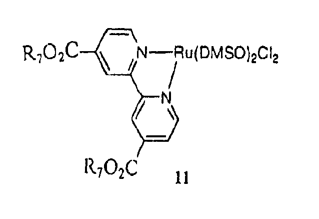

13. Compound according to formula 10 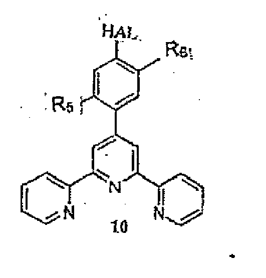 where Hal represents a halogen atom, preferentially a Br atom and where R₅ and R₆, identical or different, are selected from the alkyl groups having 1 to 24C, preferentially 1 to 12C, and the alcoxy groups having 1 to 24C, preferentially 1 to 12C.

14. Semiconductor inorganic-organic hybrid material P-N comprising a porous oxide ceramic substrate to which a complex of formula (I) according to any one of claims 1 to 10 is chemically grafted.

15. Material according to claim 14, wherein the porous oxide ceramic is selected from ceramics based on transition metals selected from Ti, V, Cr, Mn, Fe, Co, Ni, Cu, Zn, Y, Zr, Nb, Mo, Ru, Rh, Pd, Ag, Cd, Hf, Ta, W, Re, Os, Ir, Pt or based on lanthanides, such as La, Ce, Pr, Nd, Sm, Eu, Gd, Tb, Dy, Er, and Yb or based on IIIA group elements from the periodic table selected from Al, Ga, In and Tl or based on IVA elements of the periodic table selected from Si, Ge, Sn and Pb or based on VIA group elements from the periodic table selected from Se and Te.

16. Semiconductor material according to any one of claims 14 and 15, wherein the porous oxide ceramic is a mesoporous ceramic.

17. Semiconductor material according to claim 16, wherein the mesoporous ceramic is mesostructured.

18. Semiconductor material according to any one of claims 14 to 17, wherein the ceramic is titanium dioxide TiO₂.

19. Method for preparing a semiconductor inorganic-organic hybrid material according to any one of claims 14 to 18, wherein a semiconductive porous oxide ceramic is impregnated with an organic solution having one or several complexes according to formula (I).

20. Photovoltaic cell comprising:
- a first current collecting electrode (referred to as "working electrode");
- a second electrode (referred to as "working counter-electrode");
- a semiconductive zone consisting of a material as defined according to any one of claims 14 to 18, said zone being arranged between said first electrode and said second electrode.

## Patentansprüche

1. Komplex der Formel (I) 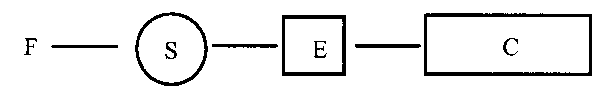 worin:
- F eine oder mehrere, zum chemischen Binden an ein Substrat aus halbleitender, poröser Oxidkeramik befähigte Gruppen darstellt;
- S eine Sensibilisatorgruppe einer halbleitenden, porösen Oxidkeramik darstellt;
- C ein elektrisch leitfähiges Polymer ist;
- E eine entkonjugierende ("deconjuguant") Abstandsgruppe ist, die das elektrische Isolieren des Sensibilisators (S) von dem elektrischen leitfähigen Polymer (C) gestattet.

2. Komplex gemäß Anspruch 1, bei dem die halbleitende, poröse Oxidkeramik eine halbleitende Keramik des n- oder p-Typs mit einem breiten verbotenen Band ist.

3. Komplex gemäß einem der vorangehenden Ansprüche, bei dem die zum chemischen Binden an ein Substrat aus halbleitender, poröser Oxidkeramik befähigte Gruppe oder Gruppen F Folgendes sind:
- COOR¹, wobei R¹ ein Wasserstoffatom. eine 1 bis 30 Kohlenstoffatome umfassende Alkylgruppe oder eine Phenylgruppe darstellt;
- COCl;
- COCH₂CO-R¹, wobei R¹ ein Wasserstoffatom, eine 1 bis 30 Kohlenstoffatome umfassende Alkylgruppe oder eine Phenylgruppe darstellt;
- PO(OH)₂, -PO(OR²)(OH) oder -PO(OR²)(OR³), wobei R² und R³, die gleich oder verschieden sind, eine 1 bis 30 Kohlenstoffatome umfassende Alkylgruppe oder eine Phenylgruppe darstellen;
- CO(NHOH);
- M(OR⁴)ₘ₋ₓZₓ, wobei x eine ganze Zahl von 1 bis (m-1) ist, M ein Metall oder ein Metalloid ist, m ein Oxidationszustand von M ist, R⁴ ein Wasserstoffatom, eine 1 bis 30 Kohlenstoffatome umfassende Alkylgruppe, eine Phenylgruppe, ein einwertiges Metallkation oder eine Gruppe der Formel N⁺R¹₄, wobei R¹ ein Wasserstoffatom. eine 1 bis 30 Kohlenstoffatome umfassende Alkylgruppe oder eine Phenylgruppe darstellt, und Z ein Wasserstoffatom. eine 1 bis 30 Kohlenstoffatome umfassende Alkylgruppe, eine Phenylgruppe oder ein Halogenatom darstellt;
- SO₃M', wobei M' ein Wasserstoffatom, ein einwertiges Metallkation oder eine Gruppe der Formel N⁺R¹₄, wobei R¹ ein Wasserstoffatom. eine 1 bis 30 Kohlenstoffatome umfassende Alkylgruppe oder eine Phenylgruppe darstellt;
- B(OM')₂, wobei M' ein Wasserstoffatom, ein einwertiges Metallkation oder eine Gruppe der Formel N⁺R¹₄, wobei R¹ ein Wasserstoffatom. eine 1 bis 30 Kohlenstoffatome umfassende Alkylgruppe oder eine Phenylgruppe darstellt;
- OH
und deren Kombinationen.

4. Komplex gemäß einem der vorangehenden Ansprüche, bei dem die Sensibilisatorgruppe S aus Polypyridinkomplexen mit einem Übergangsmetall und organischen Kationen wie etwa Phthalocyaninen, Cumarinen und Cyaninen ausgewählt ist.

5. Komplex gemäß Anspruch 4, bei dem die Sensibilisatorgruppe eine Gruppe der Formel 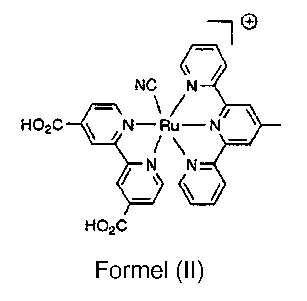 oder der Formel 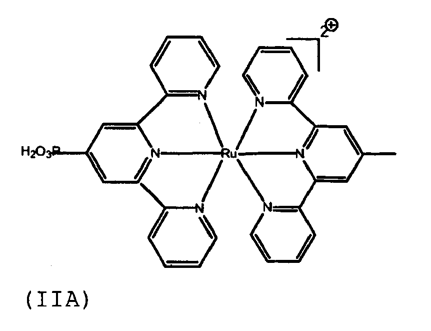 ist.

6. Komplex gemäß einem der vorangehenden Ansprüche, bei dem das elektrisch leitfähige Polymer C aus Poly(acetylen)en, Poly(p-phenylen)en, Poly(p-phenylenvinylen)en, Poly(p-phenylensulfid)en, Poly(pyrrol)en, Poly(thiophen)en, Poly(alkylthiophen)en, Poly(dialkylthiophen)en, Poly(furan)en, Poly(alkoxythiophen)en, Poly(azulen)en, Poly(azin)en, Poly(anilin)en, Poly(cyanphenylenvinylen)en, Poly(p-pyridylvinylen)en, Poly(dioxythiophen)en ("PEDOT") und deren Gemischen und/oder Kombinationen und/oder Copolymeren ausgewählt ist.

7. Komplex gemäß Anspruch 6, bei dem das elektrisch leitfähige Polymer C ein regioreguläres Polymer ist.

8. Komplex gemäß Anspruch 7, bei dem das elektrisch leitfähige Polymer aus den folgenden Polymeren ausgewählt ist: 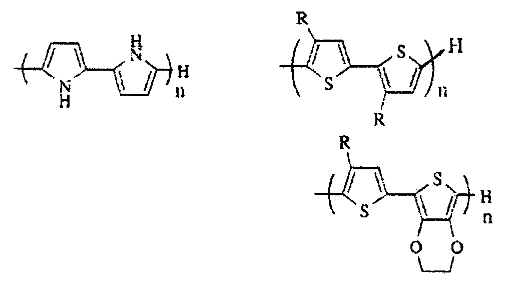 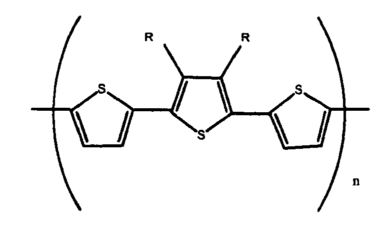 worin n eine ganze Zahl von 1 bis 1000, vorzugsweise von 5 bis 100 darstellt und R eine aus C₁₋₂₄-, vorzugsweise C₁₋₁₂-Alkylgruppen, wobei R zum Beispiel eine n-Octylgruppe ist, und C₁₋₂₄-, vorzugsweise C₁₋₁₂-Alkoxygruppen ausgewählte Gruppe darstellt.

9. Komplex gemäß einem der vorangehenden Ansprüche, bei dem die entkonjugierende ("déconjuguant") Abstandsgruppe E ausgewählt ist aus den Gruppen: 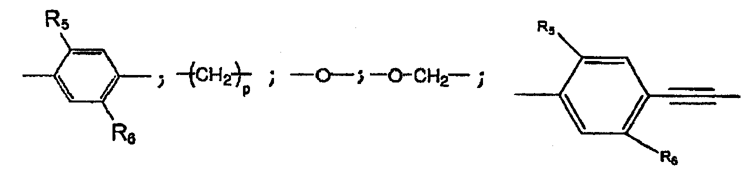 worin R₅ und R₆, die gleich oder verschieden sind, aus C₁₋₂₄-, vorzugsweise C₁₋₁₂-Alkylgruppen und C₁₋₂₄-, vorzugsweise C₁₋₁₂-Alkoxygruppen ausgewählt sind und p eine ganze Zahl von 1 bis 20, vorzugsweise 1 bis 4 ist, und

10. Komplex gemäß Anspruch 1, der der folgenden Formel (III) oder (IIIA) entspricht: 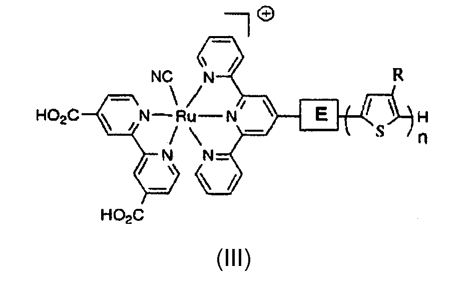 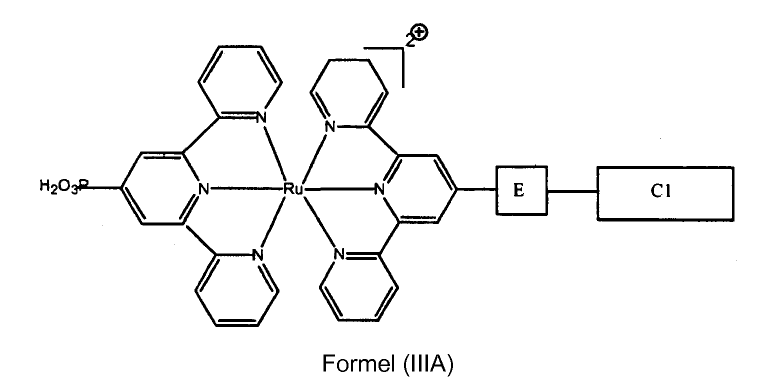 worin n eine ganze Zahl von 1 bis 1000, vorzugsweise von 5 bis 100 darstellt; R eine C₁₋₂₄-, vorzugsweise C₁₋₁₂-Alkylgruppe oder eine C₁₋₂₄-, vorzugsweise C₁₋₁₂-Alkoxygruppe darstellt; E aus den Gruppen 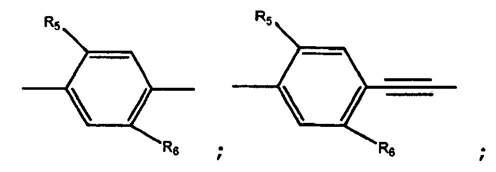 und -(CH₂)₂-
ausgewählt ist; R₅ und R₆, die gleich oder verschieden sind, aus C₁₋₂₄-, vorzugsweise C₁₋₁₂-Alkylgruppen und C₁₋₂₄-, vorzugsweise C₁₋₁₂-Alkoxygruppen ausgewählt sind und C1 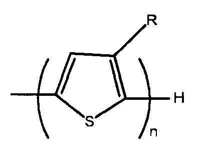 oder 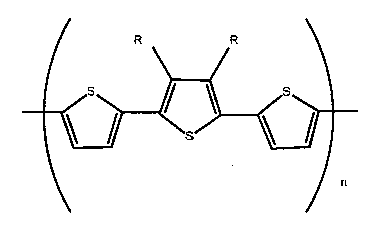 darstellt.

11. Verfahren zur Herstellung eines Komplexes der Formel (III) gemäß Anspruch 10, worin E -(CH₂)₂- oder 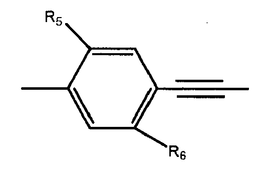 darstellt, bei dem die folgenden, aufeinanderfolgenden Schritte durchgeführt werden:
a) - eine Verbindung der folgenden Formel 1 oder eine Verbindung der folgenden 12 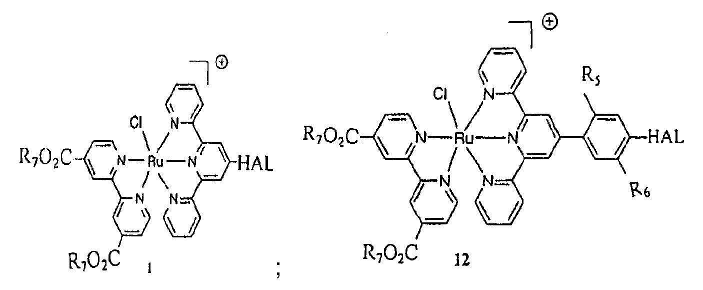 wird mit einer Verbindung der folgenden Formel 2 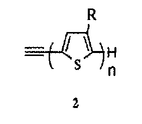 worin n eine ganze Zahl von 1 bis 1000, vorzugsweise von 5 bis 100 darstellt und R, R₅, R₆ und R₇, die gleich oder verschieden sind, eine C₁₋₂₄-, vorzugsweise C₄₋₁₂- für R und vorzugsweise C₁₋₁₂-Alkylgruppe für R₅, R₆ und R₇ oder eine C₁₋₂₄-, vorzugsweise C₄₋₁₂- für R und vorzugsweise C₁₋₁₂-Alkoxygruppe für R₅, R₆ und R₇, weiter bevorzugt R₇ eine Ethylgruppe ist, und HAL ein Halogenatom, vorzugsweise ein Br-Atom darstellt, nach einer SONOGASHIRA-Reaktion in einem DMF/THF-Gemisch in Gegenwart eines Kupferiodid, [1,1'-Bis(diphenylphosphino)ferrocen]dichlorpalladium(II) und Triethylamin umfassenden katalytischen Systems unter Erhalten einer Verbindung der folgenden Formel 3 beziehungsweise einer Verbindung der folgenden Formel 13 umgesetzt: 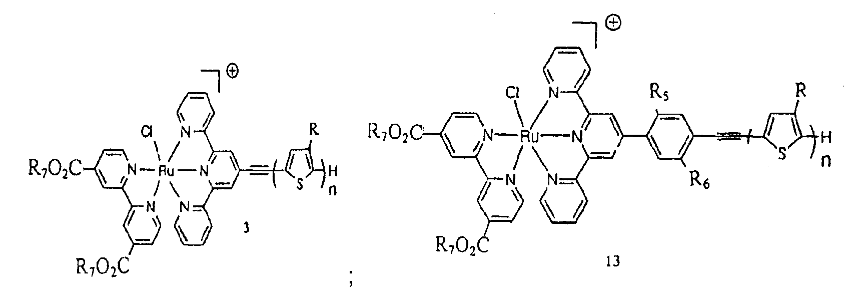
b) - die Verbindung der Formel 3 wird mit Wasserstoff in THF in Gegenwart von Palladiumauf-Kohle unter Erhalten einer Verbindung der folgenden Formel 4 umgesetzt: 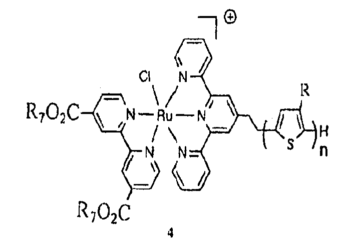
c) - die Verbindung der Formel 4 oder die Verbindung der Formel 13 wird in einem THF/H₂O-Gemisch mit KCN/LiOH unter Erhalten einer Verbindung der folgenden Formel 5 oder der folgenden Formel 14 umgesetzt:

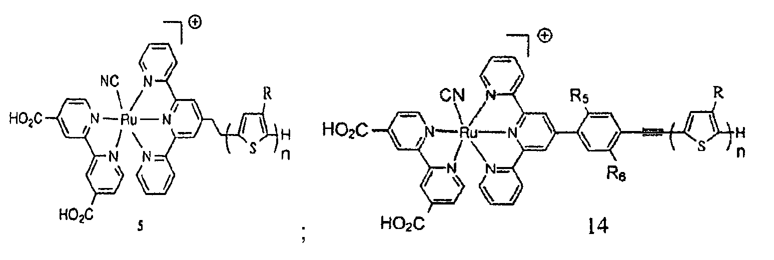

12. Verfahren gemäß Anspruch 11, bei dem die Verbindung der Formel 12 durch Reaktion einer Verbindung der Formel 10 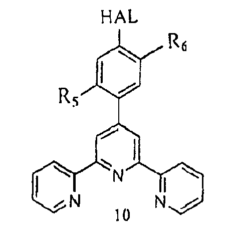 worin HAL ein Halogenatom, vorzugsweise ein Br-Atom darstellt, mit einer Verbindung der Formel 11 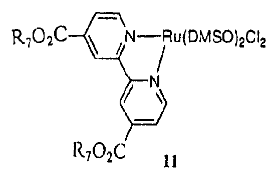 hergestellt wird.

13. Verbindung der Formel 10 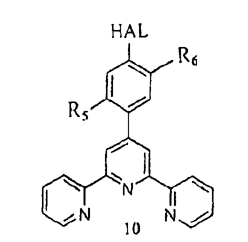 worin HAL ein Halogenatom, vorzugsweise ein Br-Atom darstellt und
R₅ und R₆, die gleich oder verschieden sind, aus C₁₋₂₄-, vorzugsweise C₁₋₁₂-Alkylgruppen und C₁₋₂₄-, vorzugsweise C₁₋₁₂-Alkoxygruppen ausgewählt sind.

14. Anorganisch-organisches p-n-Hybridhalbleitermaterial umfassend ein Substrat aus poröser Oxidkeramik, an das ein Komplex der Formel (I) gemäß einem der Ansprüche 1 bis 10 chemisch gebunden ist.

15. Material gemäß Anspruch 14, bei dem die poröse Oxidkeramik aus Keramiken auf der Grundlage aus Ti, V, Cr, Mn, Fe, Co, Ni, Cu, Zn, Y, Zr, Nb, Mo, Ru, Rh, Pd, Ag, Cd, Hf, Ta, W, Re, Os, Ir, Pt ausgewählter Übergangsmetalle oder auf der Grundlage von Lanthaniden wie etwa La, Ce, Pr, Nd, Sm, Eu, Gd, Tb, Dy, Er und Yb oder auf der Grundlage von aus Al, Ga, In und TI ausgewählten Elementen der Gruppe IIIA des Periodensystems oder auf der Grundlage von aus Si, Ge, Sn und Pb ausgewählten Elementen der Gruppe IVA des Periodensystems oder auf der Grundlage von aus Se und Te ausgewählten Elementen der Gruppe VIA des Periodensystems ausgewählt ist.

16. Halbleitermaterial gemäß einem der Ansprüche 14 und 15, bei dem die poröse Oxidkeramik eine mesoporöse Keramik ist.

17. Halbleitermaterial gemäß Anspruch 16, bei dem die mesoporöse Keramik mesostrukturiert ist.

18. Halbleitermaterial gemäß einem der Ansprüche 14 bis 17, bei dem die Keramik Titandioxid TiO₂ ist.

19. Verfahren zur Herstellung des anorganisch-organischen Hybridhalbleitermaterials gemäß einem der Ansprüche 14 bis 18, bei dem eine halbleitende, poröse Oxidkeramik mit einer organischen Lösung getränkt wird, die einen oder mehrere Komplexe der Formel (I) aufweist.

20. Photovoltaikzelle umfassend
- eine erste Stromsammelelektrode ("Arbeitselektrode" genannt);
- eine zweite Elektrode ("Arbeitsgegenelektrode" genannt);
- eine halbleitende Zone, die sich aus einem wie gemäß einem der Ansprüche 14 bis 18 definierten Material zusammensetzt, wobei sich die besagte Zone zwischen der besagten ersten Elektrode und der besagten zweite Elektrode befindet.
